# EUROPEAN PATENT SPECIFICATION

(11) **EP 1 644 039 B1**
(45) Date of publication and mention of the grant of the patent: **01.10.2014**
(21) Application number: 04754498.6
(22) Date of filing: 04.06.2004
(51) Int. Cl.: A61K 39/395, C07K 16/36, A61P 7/02

(54) **COMPOSITIONS AND METHODS FOR TREATING COAGULATION RELATED DISORDERS**
ZUSAMMENSETZUNGEN UND VERFAHREN ZUR BEHANDLUNG VON GERINNUNGSSTÖRUNGEN
COMPOSITIONS ET PROCEDES POUR LE TRAITEMENT DES TROUBLES LIES A LA COAGULATION

(30) Priority: 19.06.2003 US 480254 P; 22.01.2004 US 538892 P
(43) Date of publication of application: 12.04.2006
(73) Proprietor: Genentech, Inc., South San Francisco, CA 94080 (US)
(72) Inventor: JIAO, Jin-an, Sioux Falls, SD 57106 (US); WONG, Hing, C., Weston, FL 33332 (US); EGAN, Jack, O., Plantation, FL 33324 (US)
(74) Representative: Evenson, Jane Harriet
(86) International application number: PCT/US2004/017900
(87) International publication number: WO 2005/004793

(56) References cited:
- WO-A-03/037911
- WO-A1-03/029295
- US-A- 5 223 427
- US-A- 5 986 065
- US-B2- 6 703 494
- WELTY-WOLF KE ET AL: "Coagulation blockade prevents sepsis-induced respiratory and renal failure in baboons" AMERICAN JOURNAL OF RESPIRATORY AND CRITICAL CARE MEDICINE, AMERICAN LUNG ASSOCIATION, NEW YORK, NY, US, vol. 164, no. 10, November 2001 (2001-11), pages 1988-1996, XP002902640 ISSN: 1073-449X
- ESMON C T: "Role of coagulation inhibitors in inflammation." THROMBOSIS AND HAEMOSTASIS JUL 2001, vol. 86, no. 1, July 2001 (2001-07), pages 51-56, XP002431858 ISSN: 0340-6245
- WELTY-WOLF K E ET AL: "Tissue Factor in Experimental Acute Lung Injury" SEMINARS IN HEMATOLOGY, PHILADELPHIA, PA, US, vol. 38, no. 4, SUPPL 12, October 2001 (2001-10), pages 35-38, XP009046977 ISSN: 0037-1963
- MILLER DEBRA L ET AL: "Extrinsic coagulation blockade attenuates lung injury and proinflammatory cytokine release after intratracheal lipopolysaccharide" AMERICAN JOURNAL OF RESPIRATORY CELL AND MOLECULAR BIOLOGY, AMERICAN LUNG ASSOCIATION, NEW YORK, NY, US, vol. 26, no. 6, June 2002 (2002-06), pages 650-658, XP002337200 ISSN: 1044-1549
- ISHIHARA KATSUHIKO ET AL: "IL-6 in autoimmune disease and chronic inflammatory proliferative disease" CYTOKINE AND GROWTH FACTOR REVIEWS, vol. 13, no. 4-5, August 2002 (2002-08), pages 357-368, XP002431859 ISSN: 1359-6101
- BOKAREWA M I ET AL: "Intra-articular tissue factor/factor VII complex induces chronic arthritis" INFLAMMATION RESEARCH, vol. 51, no. 9, September 2002 (2002-09), pages 471-477, XP002431860 ISSN: 1023-3830
- BUSSO NATHALIE ET AL: "Role of the tissue factor pathway in synovial inflammation." ARTHRITIS AND RHEUMATISM MAR 2003, vol. 48, no. 3, March 2003 (2003-03), pages 651-659, XP002431861 ISSN: 0004-3591
- ERLICH J H ET AL: "Tissue factor initiates glomerular fibrin deposition and promotes major histocompatibility complex class II expression in crescentic glomerulonephritis." THE AMERICAN JOURNAL OF PATHOLOGY MAR 1997, vol. 150, no. 3, March 1997 (1997-03), pages 873-880, XP002431862 ISSN: 0002-9440
- ASADULLAH K ET AL: "The pathophysiological role of cytokines in psoriasis." DRUGS OF TODAY (BARCELONA, SPAIN : 1998) DEC 1999, vol. 35, no. 12, December 1999 (1999-12), pages 913-924, XP002431863 ISSN: 1699-3993
- MAIMONE D ET AL: "T cell lymphokine-induced secretion of cytokines by monocytes from patients with multiple sclerosis." CELLULAR IMMUNOLOGY JAN 1993, vol. 146, no. 1, January 1993 (1993-01), pages 96-106, XP002431864 ISSN: 0008-8749
- SKOPOULI F N ET AL: "Cytokines in Sjögren's syndrome" ANNALES DE MEDECINE INTERNE, MASSON, PARIS, FR, vol. 146, no. 4, 1995, pages 219-222, XP000886636 ISSN: 0003-410X
- MORE L ET AL: "Immunohistochemical study of tissue factor expression in normal intestine and idiopathic inflammatory bowel disease." JOURNAL OF CLINICAL PATHOLOGY AUG 1993, vol. 46, no. 8, August 1993 (1993-08), pages 703-708, XP002431865 ISSN: 0021-9746
- CARRAWAY MARTHA SUE ET AL: "Blockade of tissue factor: treatment for organ injury in established sepsis." AMERICAN JOURNAL OF RESPIRATORY AND CRITICAL CARE MEDICINE 1 MAY 2003, vol. 167, no. 9, 1 May 2003 (2003-05-01), pages 1200-1209, XP002431866 ISSN: 1073-449X
- HOUSTON D S: "Tissue factor - a therapeutic target for thrombotic disorders" EXPERT OPINION ON THERAPEUTIC TARGETS, ASHLEY PUBLICATIONS, LONDON, GB, vol. 6, no. 2, April 2002 (2002-04), pages 159-174, XP009035643 ISSN: 1472-8222

### SEQUENCE LISTING

(1) GENERAL INFORMATION
   (i) APPLICANT:
   (ii) TITLE OF THE INVENTION:
   (iii) NUMBER OF SEQUENCES: 26
   (iv) CORRESPONDENCE ADDRESS:
      (A) ADDRESSEE:
      (B) STREET:
      (C) CITY:
      (D) STATE:
      (E) COUNTRY:
      (F) ZIP:
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ Version 1.5
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME:
      (B) REGISTRATION NUMBER:
      (C) REFERENCE/DOCKET NUMBER:
   (ix) TELECOMMUNICATION INFORMATION:
      (A) TELEPHONE:
      (B) TELEFAX:
      (C) TELEX:
   (2) INFORMATION FOR SEQ ID NO:1:
   (i) SEQUENCE CHARACTERISTICS:
      (A) LENGTH: 321 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:1:
   GACATTCAGA TGACCCAGTC TCCTGCCTCC CAGTCTGCAT CTCTGGGAGA AAGTGTCACC
   60
   ATCACATGCC TGGCAAGTCA GACCATTGAT ACATGGTTAG CATGGTATCA GCAGAAACCA
   120
   GGGAAATCTC CTCAGCTCCT GATTTATGCT GCCACCAACT TGGCAGATGG GGTCCCATCA
   180
   AGGTTCAGTG GCAGTGGATC TGGCACAAAA TTTTCTTTCA AGATCAGCAG CCTACAGGCT
   240
   GAAGATTTTG TAAATTATTA CTGTCAACAA GTTTACAGTT CTCCATTCAC GTTCGGTGCT
   300
   GGGACCAAGC TGGAGCTGAA A
   321
(2) INFORMATION FOR SEQ ID NO:2:
   (i) SEQUENCE CHARACTERISTICS:
      (A) LENGTH: 107 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:2:
   Asp Ile Gln Met Thr Gln Ser Pro Ala Ser Gln Ser Ala Ser Leu Gly
    1               5                  10                  15
   Glu Ser Val Thr Ile Thr Cys Leu Ala Ser Gln Thr Ile Asp Thr Trp
               20                  25                  30
   Leu Ala Trp Tyr Gln Gln Lys Pro Gly Lys Ser Pro Gln Leu Leu Ile
           35                  40                  45
   Tyr Ala Ala Thr Asn Leu Ala Asp Gly Val Pro Ser Arg Phe Ser Gly
       50                  55                  60
   Ser Gly Ser Gly Thr Lys Phe Ser Phe Lys Ile Ser Ser Leu Gln Ala
   65                  70                  75                  80
   Glu Asp Phe Val Asn Tyr Tyr Cys Gln Gln Val Tyr Ser Ser Pro Phe
                   85                  90                  95
   Thr Phe Gly Ala Gly Thr Lys Leu Glu Leu Lys
               100                 105
(2) INFORMATION FOR SEQ ID NO:3:
   (i) SEQUENCE CHARACTERISTICS:
      (A) LENGTH: 351 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:3:
    GAGATCCAGC TGCAGCAGTC TGGACCTGAG CTGGTGAAGC CTGGGGCTTC AGTGCAGGTA
   60
    TCCTGCAAGA CTTCTGGTTA CTCATTCACT GACTACAACG TGTACTGGGT GAGGCAGAGC
   120
    CATGGAAAGA GCCTTGAGTG GATTGGATAT ATTGATCCTT ACAATGGTAT TACTATCTAC
   180
    GACCAGAACT TCAAGGGCAA GGCCACATTG ACTGTTGACA AGTCTTCCAC CACAGCCTTC
   240
    ATGCATCTCA ACAGCCTGAC ATCTGACGAC TCTGCAGTTT ATTTCTGTGC AAGAGATGTG
   300
    ACTACGGCCC TTGACTTCTG GGGCCAAGGC ACCACTCTCA CAGTCTCCTC A
   351
(2) INFORMATION FOR SEQ ID NO:4:
   (i) SEQUENCE CHARACTERISTICS:
      (A) LENGTH: 117 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:4:
   Glu Ile Gln Leu Gln Gln Ser Gly Pro Glu Leu Val Lys Pro Gly Ala
    1               5                  10                  15
   Ser Val Gln Val Ser Cys Lys Thr Xaa Gly Tyr Ser Phe Thr Asp Tyr
               20                  25                  30
   Asn Val Tyr Trp Val Arg Gln Ser His Gly Lys Ser Leu Glu Trp Ile
           35                  40                  45
   Gly Tyr Ile Asp Pro Tyr Asn Gly Ile Thr Ile Tyr Asp Gln Asn Phe
       50                  55                  60
   Lys Gly Lys Ala Thr Leu Thr Val Asp Lys Ser Ser Thr Thr Ala Phe
   65                  70                  75                  80
   Met His Leu Asn Ser Leu Thr Ser Asp Asp Ser Ala Val Tyr Phe Cys
                   85                  90                  95
   Ala Arg Asp Val Thr Thr Ala Leu Asp Phe Trp Gly Gln Gly Thr Thr
               100                 105                 110
   Leu Thr Val Ser Ser
           115
(2) INFORMATION FOR SEQ ID NO:5:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:5:
   Leu Ala Ser Gln Thr Ile Asp
    1               5
(2) INFORMATION FOR SEQ ID NO:6:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:6:
   Ala Ala Thr Asn Leu Ala Asp
   1 5
(2) INFORMATION FOR SEQ ID NO:7:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:7:
   Gln Gln Val Tyr Ser Ser Pro Phe Thr
   1 5
(2) INFORMATION FOR SEQ ID NO:8:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:8:
   Thr Asp Tyr Asn Val Tyr
   1 5
(2) INFORMATION FOR SEQ ID NO:9:
   (i) SEQUENCE CHARACTERISTICS:
      (A) LENGTH: 17 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:9:
   Tyr Ile Asp Pro Tyr Asn Gly Ile Thr Ile Tyr Asp Gln Asn Phe Lys
   1 5 10 15
   Gly
(2) INFORMATION FOR SEQ ID NO:10:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTISENSE: NO
   (v) FRAGMENT TYPE: N-terminal
   (vi) ORIGINAL SOURCE:
   (xi) SEQUENCE DESCRIPTION: SEQ ID NO:10:
   Asp Val Thr Thr Ala Leu Asp Phe
   1 5
(2) INFORMATION FOR SEQ ID NO:11:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:11:
   CTGGCAAGTC AGACCATTGA T
   21
(2) INFORMATION FOR SEQ ID NO:12:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:12:
   GCTGCCACCA ACTTGGCAGA T
   21
(2) INFORMATION FOR SEQ ID NO:13:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:13:
   CAACAAGTTT ACAGTTCTCC ATTCACGT
   28
(2) INFORMATION FOR SEQ ID NO:14:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:14:
   ACTGACTACA ACGTGTAC
   18
(2) INFORMATION FOR SEQ ID NO:15:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:15:
   TATATTGATC CTTACAATGG TATTACTATC TACGACCAGA ACTTCAAGGG C
   51
(2) INFORMATION FOR SEQ ID NO:16:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:16:
   GATGTGACTA CGGCCCTTGA CTTC
   24
(2) INFORMATION FOR SEQ ID NO:17:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE. NO
   (v) FRAGMENT TYPE:
   (vi) ORIGINAL SOURCE:
   (xi) SEQUENCE DESCRIPTION: SEQ ID NO:17:
   GCACCTCCAG ATGTTAACTG CTC
   23
(2) INFORMATION FOR SEQ ID NO:18:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE : DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:18:
   GAARTAVCCC TTGACCAGGC
   20
(2) INFORMATION FOR SEQ ID NO:19:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:19:
   GGAGGCGGCG GTTCTGACAT TGTGMTGWCM CARTC
   35
(2) INFORMATION FOR SEQ ID NO:20:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:20:
   ATTTCAGGCC CAGCCGGCCA TGGCCGARGT YCARCTKCAR CARYC
   45
(2) INFORMATION FOR SEQ ID NO:21;
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE : DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:21:
   CCCGGGCCAC CATGKCCCCW RCTCAGYTYC TKG
   33
(2) INFORMATION FOR SEQ ID NO:22:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi). ORIGINAL SOURCE:
   (xi) SEQUENCE DESCRIPTION: SEQ ID NO:22:
   CCCGGGCCAC CATGGRATGS AGCTGKGTMA TSCTC
   35
(2) INFORMATION FOR SEQ ID NO:23:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE : DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:23:
   ATATACTCGC GACAGCTACA GGTGTCCACT CCGAGATCCA GCTGCAGCAG TC
   52
(2) INFORMATION FOR SEQ ID NO:24:
   (i) SEQUENCE CHARACTERISTICS:
      (A) LENGTH: 31 base pairs
      (B) TYPE: nucleic acid
      (C) STRANDEDNESS : single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:24:
   GACCTGAATT CTAAGGAGAC TGTGAGAGTG G
   31
(2) INFORMATION FOR SEQ ID NO:25:
   (i) SEQUENCE CHARACTERISTICS:
      (A) LENGTH: 29 base pairs
      (B) TYPE: nucleic acid
      (C) STRANDEDNESS: single ,
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:25:
   TTAATTGATA TCCAGATGAC CCAGTCTCC
   29
(2) INFORMATION FOR SEQ ID NO:26:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: DNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:26:
   TAATCGTTCG AAAAGTGTAC TTACGTTTCA GCTCCAGCTT GGTCC
   45

## Description

### FIELD OF THE DISCLOSURE

The present disclosure features compositions and methods for preventing or treating disorders that relate to undesired activation of blood coagulation. In some instances, the coagulation contributes to certain inflammatory diseases and related disorders. In one aspect, the disclosure provides methods for treating such disorders by administering a therapeutically effective amount of a chimeric or humanized antibody that binds tissue factor (TF) specifically. The disclosure has a wide spectrum of important applications including use in the prevention or treatment of inflammation including sepsis and arthritis.

### BACKGROUND OF THE INVENTION

There is increasing recognition of relationship between coagulation and inflammation. For instance, certain coagulation factors are thought to activate pro-inflammatory cells and elicit inflammatory responses. On the other hand, some pro-inflammatory cytokines have been reported to induce TF expression and generate coagulation factors. Studies in certain primates support existence of the relationship by showing that certain anticoagulants reduce inflammation. See F. B. Taylor Jr. et al., J. Clin. Invest. 79:918-925 (1987); M. Levi et al., J. Clin. Invest. 93:114-120 (1994); and M. C. Minnema et al., Blood 95:1117-23 (2000).

Much has been reported about blood coagulation. For instance, thrombin is a blood protein that is believed to provide a link between coagulation and inflammation. Most thrombin is generated by TF-initiation of the coagulation cascade. Other key coagulation factors include Factor VIIa and Factor Xa. Thrombin is thought to occupy multiple roles in pro-coagulant, anticoagulant, inflammatory, and mitogenic responses. See generally L. Styer, Biochemistry, 3rd Ed., W.H. Freeman Co., New York; and A.G. Gilman et al., in The Pharmacological Basis of Therapeutics, 8th Ed., McGraw-Hill Inc., New York.

Certain infectious agents are thought to disturb the TF-initiated coagulation cascade. Fortunately, localized activation of the coagulation cascade often keeps the disturbance in check. Sometimes however, aberrant TF expression leads to serious and potentially life-threatening thrombotic disorders. For example, it has been suggested that bacterial induction of TF expression can lead to sepsis, disseminated intravascular coagulation (DIC), widespread fibrin deposition, and other complications. The increase in TF expression is thought to be an important factor in facilitating progression of these disorders. An example of progression of an inflammatory disorder is the progression of acute lung injury (ALI) to acute respiratory distress syndrome (ARDS) followed by progressive injury involving additional organ systems, such as the kidneys, leading to progressively more severe forms of sepsis. See Welty-Wolf, K. et al. Am. J. Respir. Crit. Care Med.164:1988 (2001), for instance.

There have been attempts to understand coagulation related disorders such as sepsis.

For instance, sepsis is a term that has used to characterize a spectrum of clinical conditions facilitated by a host immune response to infection, trauma, or both. Sepsis has been characterized as an uncontrolled cascade of blood coagulation, fibrinolysis, and inflammation. At certain steps in the cascade, an auto-amplification process contributes to an acceleration of coagulation abnormalities, undesired inflammation, and endothelial injury. The amplification process is the result of inflammatory cytokines up-regulating the expression of TF on cells such as endothelial cells and monocytes, resulting in increased activation of the coagulation cascade, which in turn results in the activation of PAR receptors and the up-regulation of the production of inflammatory cytokines. See Osterud, B. et al., Thromobsis Hemost. 83:861 (2000); Mechtcheriakova, D. et al., FASEB J. 15:230 (2001); Shen, B.-Q. et al., J. Biol. Chem. 276:5281 (2001).

More specifically, sepsis has been characterized by systemic activation of inflammation and blood coagulation. Fibrinolyisis can be suppressed. Some research has pointed to a hemostatic imbalance that is thought to promote e.g., DIC and microvascular thrombosis. These and related indication are believed to impact normal organ function which can lead to death.

Sepsis and related disorders continue to be linked to patient contact with hospitals and out-patient clinics. Accordingly, managing these disorders is a prime concern of many health administrators, clinicians and insurers.

There are reports that undesired activation of blood coagulation is a key feature of certain inflammatory diseases. For example, extravascular fibrin deposition has been found in the autoimmune lesions of patients with rheumatoid arthritis (RA), glomerulonephritis, multiple sclerosis, psoriasis, Sjogren's syndrome and inflammatory bowel disease. See Weinberg, et al., Arthritis Rheum. 34:996-1005 (1991); Wakefield, et al., J Clin Patliol 47:129-133 (1994); Schoph, et al., Arch Dermatol Res 285:305-309 (1993); Zeher, et al., Clin Immunol Immunopathol 71:149-155 (1994); More, et al., J Clin Pathol 46:703-708 (1993).

Moreover, elevated TF levels are thought to be associated with systemic lupus erythematosus. See Segal, et al., J Rheumatol 27:2827-2832 (2000). See also Nakano, et al., Clin Exp Rheumatol 17:161-170 (1999); Morris, et al., Ann Rheum Dis 53:72-79 (1994); Furmaniak-Kazmierczak, et al., J Clin Invest 94:472-480 (1994); Bokarewa, et al., Inflamm Res 51:471-477 (2002).

There have been suggestions that certain inflammatory conditions may be treated by blocking thrombosis. See R. Gordon et al., The New England Journal of Medicine 344:699-709, 759-762 (2001). Certain anti-TF antibodies have been reported to be of some help in reducing some types of inflammation. See Levi, M. *et al., supra.*

There have been efforts to develop antibodies that bind blood coagulation factors.

For instance, U.S. Pat. Nos. 5,986,065 and 6,555,319 to Wong, H. et al. and PCT/US98/04644 (WO 98/40408) disclose a variety of such antibodies. Specifically provided are murine antibodies, chimeric antibody derivatives and fragments thereof with significant binding affinity and specificity for tissue factor (TF). Use of chimeric antigen binding molecules are believed to reduce risk of an undesired immune response in human patients. See also S. L.Morrison and V. Oi, Adv. Immunol. 44:65 (1989) (reporting methods of making human-mouse chimeric antibodies).

A variety of approaches have been used to make antibodies more immunologically acceptable to humans. Some use recombinant DNA technologies. For instance, one strategy has been to clone and modify non-human antibodies to more closely resemble human antibodies. Collectively, such antibodies have been referred to as "humanized". See U.S Pat. Nos. 5,766,886 to Studnicka et al.*;* 5,693,762 to Queen et al.*;* 5,985,279 to Waldeman et al.*;* 5,225,539 to Winter; 5,639,641 to Pedersen, et al.*;* and references cited therein for methods of making and using humanized antibodies.

Additional strategies for making humanized antibodies have been reported. See E. Padlan Mol. Immunol. 28:489 (1991); Jones et al., Nature 321:522-525 (1986); Junghans *et al., supra;* and Roguska, et al., PNAS (USA) 91:969 (1994). See also published U.S Patent Applications 2003/0109860 A1 and 2003/0082636.

It is unclear if prior antibodies are robust enough to block or reduce undesired activation of blood coagulation. More specifically, it is unclear if such antibodies are potent enough to prevent or treat sepsis and inflammatory diseases such as arthritis.

### SUMMARY OF THE INVENTION

The present invention is as defined by the claims.

The present disciosure features compositions and methods for preventing or treating disorders relating to undesired activation of blood coagulation. In one aspect, the disclosure provides methods for preventing or treating such disorders by administering to a mammal a therapeutically effective amount of a chimeric or humanized antibody that binds tissue factor (TF). The disclosure has a wide spectrum of important applications including use in the treatment of sepsis and inflammatory diseases such as arthritis.

We have found that antibodies and antigen binding fragments thereof that specifically bind an epitope predominant to native human TF are suitable for preventing or treating disorders relating to undesired activation of blood coagulation. Preferred antibodies and fragments specifically bind native human TF and do not substantially bind non-native or denatured TF. More particular antibodies and fragments suitable for use with the present disclosure bind human TF so that at least one of Factor X (FX) and Factor IX (FIX) do not effectively bind to the TF-Factor VIIa complex. Additionally preferred antibodies and fragments reduce or block TF function, typically by reducing or blocking FX binding or gaining access to TF molecules. Further preferred antibodies and fragments suitable for use with the disclosure do not significantly inhibit or block interaction or binding between TF and Factor VIIa, or inhibit or block activity of a TF-Factor VIIa complex with respect to materials other than FX or FIX.

As discussed, it is believed that undesired activation of blood coagulation underlies sepsis and a variety of specific inflammatory diseases. More specifically, unwanted TF-mediated coagulation is thought to initiate and/or prolong such disorders in many cases. It is thus an object of the present invention to provide antibodies and fragments thereof that specifically bind TF to reduce or inactivate many if not all TF-associated functions. Such functions include, but are not limited to, blocking or inhibiting at least one of FIX and FX binding to the TF complex. Without wishing to be bound to theory, it is believed that by blocking or inhibiting binding of at least one of those factors to the TF complex, activation of unwanted blood coagulation can be reduced or in some cases eliminated. That is, by blocking or inhibiting such unwanted processes according to the disclosure, it is believed that it is possible to prevent, treat or alleviate symptoms associated with sepsis and specific inflammatory diseases.

Preferably, such antibodies and fragments thereof are chimeric or humanized and are generally suitable for use in primates and particularly human patients in need of treatment.

There is recognition that sepsis and related conditions result from a potent and potentially life-threatening immune response to infection, trauma, or both. Typically, blood throughout the vasculature is subject to coagulation, resulting in complications such as inflammation, disseminated intravascular coagulation (DIC), clotting, and organ distress. Death can ensue in a matter of hours or less unless the condition is treated rapidly. Prior to the present invention, it was not clear if any anti-TF antibody or TF-binding fragment would be robust enough to prevent or treat sepsis and related conditions.

However, it has been found that the anti-TF binding antibodies described herein are robust enough (*i.e*., bind human TF specifically enough and with appropriate avidity) to inhibit or block unwanted activation of the coagulation cascade. It has also been found that such activity is beneficial and can be used to prevent or treat sepsis and related conditions. As discussed below, we have found that such antibodies and fragments show good attenuation of inflammation, disseminated intravascular coagulation (DIC), clotting, organ distress and related conditions in an *in vivo* animal model of human sepsis.

Such potent blocking or inhibition of the blood coagulation cascade has also been found to be highly useful in the prevention or treatment of certain inflammatory diseases. Without wishing to be bound to theory, it is believed that by using the disclosure to block or reduce undesired activation of blood coagulation, it is possible to prevent, treat or alleviate symptoms associated with one or a combination of the inflammatory diseases. Importantly, the anti-TF binding antibodies described herein have been found to be robust enough to inhibit or block unwanted activation of the coagulation cascade, thereby helping to prevent, treat or alleviate symptoms associated with arthritis and other inflammatory diseases.

Accordingly, and in one aspect, the disclosure provides a method for preventing or treating at least one of sepsis and an inflammatory disease in a mammal. In one embodiment, the method includes administering to the mammal a therapeutically effective amount of at least one humanized antibody, chimeric antibody, or fragment thereof that binds specifically to human TF to form a complex. A more preferred antibody reduces or blocks at least one of FX and FIX binding to the complex. Practice of the method is highly useful for preventing, treating, or reducing the severity of symptoms associated with sepsis and inflammatory diseases including, but not limited to, rheumatoid arthritis (RA).

The present invention provides other important uses and advantages.

For instance, we have discovered that preferred humanized antibodies, chimeric antibodies and fragments thereof desirably block at least one of FX and FIX binding to the TF-FVIIa complex. Preferably, such antibodies and fragments also inhibit or block FX or FIX activation by the complex. Surprisingly, such antibodies, when tested in the *in vivo* animal of human sepsis provided herein, are robust enough to prevent, treat or alleviate symptoms of sepsis and related complications. Also surprisingly, such preferred antibodies and fragments are robust enough to prevent, treat, or alleviate symptoms associated with particular inflammatory diseases. Importantly, preferred use of the present invention has identified and takes advantage of a particular immunological target (epitope) on the human TF molecule that can be exploited to prevent, treat, or alleviate symptoms associated with these indications.

The disclosure is flexible and can be used in a variety of settings in which sepsis and inflammatory diseases may be suspected or predominate.

For instance, and in one embodiment, the disclosure can be used in hospitals, clinics and other medical settings where sepsis has become a major health problem. Especially problematic has been emergence of antibiotic resistance microorganisms such as bacteria which, if present in the blood, can rapidly produce septic shock and related complications. Practice of the disclosure can be used to hold the sepsis or related condition at bay while caregivers identify an appropriate treatment protocol, or perhaps reverse the effects of sepsis. It is thus anticipated as an object of the invention to provide sepsis prevention or treatment methods in which administration of the antibodies and fragments disclosed herein may be combined with administration of one or more antibiotics.

The disclosure finds further use in emergency medical settings (e.g., ambulance, combat) in which the prevention and treatment methods disclosed herein can be administered at the point of care. Thus in one disclosure embodiment, the sepsis or sepsis-related condition can be held under some control while the patient is transported to a hospital or clinic for evaluation and treatment.

In other disclosure embodiments, the disclosure can be used to prevent, treat or reduce symptoms associated with arthritis and especially rheumatoid arthritis. For instance, by blocking or reducing the unwanted activation of blood coagulation, it is now possible to reduce the painful inflammation, pathological tissue destruction and remodeling typically associated with arthritis and related inflammatory diseases.

In another aspect, the disclosure provides a kit for performing the methods of this disclosure. In one embodiment, the kit includes at least one humanized antibody, chimeric antibody, or fragment thereof provided herein.

The disclosure also provides a method for reducing cytokine production in a mammal. In one embodiment, the method includes administering to the mammal a therapeutically effective amount of at least one humanized antibody, chimeric antibody, or fragment thereof that binds specifically to tissue factor (TF) to form a complex. Preferably, Factor X or Factor IX binding to the complex is inhibited and the administration is sufficient to reduce the cytokine production in the mammal. Suitable humanized antibodies, chimeric antibodies and fragments thereof are disclosed above and in the discussion and examples that follow.

Further provided by the present disclosure is a method for preventing or treating a sepsis-related condition in a mammal. In one embodiment, the method includes administering to the mammal a therapeutically effective amount of at least one humanized antibody, chimeric antibody, or fragment thereof that binds specifically to tissue factor (TF) to form a complex. Preferred antibodies and fragments are described herein and include those in which Factor X or factor IX binding to the complex is inhibited. Preferably, administration is sufficient to prevent or treat the condition in the mammal.

The disclosure further provides a method for preventing or treating sepsis-induced anemia in a mammal. In one embodiment, the method includes administering to the mammal a therapeutically effective amount of at least one humanized antibody, chimeric antibody, or fragment thereof that binds specifically to tissue factor (TF) to form a complex. Preferred antibodies and fragments are disclosed herein including those in which Factor X or Factor IX binding to the complex is inhibited. Preferably, the administration is sufficient to prevent or treat the condition in the mammal.

Other aspects of the invention are discussed *infra*.

### BRIEF DESCRIPTION OF THE DRAWINGS

Figs. 1A and 1B shows the nucleic acid (SEQ ID NOS: 1 and 3) and amino acid (SEQ ID NOS: 2 and 4) sequences of light chain and heavy chain variable domains of H36.D2.B7, the murine anti-tissue factor antibody, with hypervariable regions (CDRs or Complementarity Determining Regions) underlined (single underline for nucleic acid sequences and double underline for amino acid sequences).
Fig. 2 is a drawing showing a plasmid map of humanized anti-TF IgG1 antibody expression vector (pSUN-34).
Figs. 3A-D are sequences of partially and fully humanized light chain (LC) variable domains of the anti-TF antibody. Fig. 3A shows the sequence named "LC-09" which is representative of a fully (humanized LC framework. Light chain CDR sequences of cH36 and LC-09 are shown in Figs. 3B-D.
Figs. 4A-D are drawings showing the sequences of partially and fully humanized heavy chain (HC) variable domains of the anti-TF antibody. Fig. 4A shows the sequence named "HC-08" which is representative of a fully humanized HC framework. Heavy chain CDR sequences for cH36 and HC-08 are shown in Figs. 4B-D.
Figs. 5A-B are sequences showing human constant domains in the IgG1 anti-tissue factor antibody (hOAT), with Fig. 5A showing the human kappa light chain constant domain and Fig. 5B showing the human IgG1 heavy chain constant domain. The figures show hOAT (IgG1) constant domain amino acid sequences.
Figs. 6A-B are sequences showing human constant domains in the IgG4 anti-tissue factor antibody (hFAT) with Fig. 6A showing the human kappa light chain constant domain and Fig. 6B showing the human IgG4 heavy chain constant domain.
Figs. 7A-D are graphs showing a change in plasma IL-6 and IL-8 (Figs. 7A-B) concentrations (Figs. 7A-B); or IL-1β and TNF-α concentrations (Figs. 7C-D) in rhesus monkeys following an infusion of live *E.coli* in a lethal sepsis model.
Figs. 8A-C are graphs showing that cH36 attentuates sepsis-induced acute lung injury (ALI). In the figures, AaDO₂ is in mmHg, time in hours, pulmonary system compliance (Cst) in ml/cm water, and pulmonary arterial pressure (PAM) in mmHg.
Figures. 9A-B are graphs showing Kidney Myeloperoxidase (A) and Small Bowel Wet/Dry Weight Ratio (B) in Baboons.
Figure 10A-D are graphs showing that cH36 attenuates sepsis and related conditions in baboons. cH36 attenuates sepsis-induced coagulopathy (fibrinogen in mg/DL; time in hours; partial prothrombin time (PTT) in seconds; and TAT in micrograms/L).
Figures 11A-B are graphs showing that elevations in serum IL-8 (A) and IL-6(B) are attenuated by treatment with cH36.
Figure 12 is a graph showing that elevations in bronchial alveolar levage IL-8, IL-6 and TNFRI levels attenuated by treatment with cH36.
Figures 13A-C are graphs showing mean urine output (13A), mean blood pH (13B), and serum bicarbonate levels (13C) in baboons treated with control and cH36 antibodies.

### DETAILED DESCRIPTION OF THE INVENTION

, As discussed, the disclosure provides a method for preventing, treating, or alleviating symptoms associated with sepsis or inflammatory diseases such as arthritis. Practice of the method involves administering to a mammal in need of such treatment a therapeutically effective amount of at least one of a humanized antibody, chimeric antibody, or human TF-binding fragment to prevent or treat these diseases and related conditions.

There have been efforts to understand the etiology of sepsis and conditions related to sepsis. For instance, there is recognition that early events in the sepsis cascade are triggered by the host's immune response, thereby facilitating damaging actions on the vascular endothelium. Subendothelial structures are exposed and collagenases are liberated. Endothelial cells expressing tissue factor (TF) are exposed, triggering the extrinsic pathway for activation of the coagulation cascade and accelerating the production of thrombin. Concurrently, the endothelial damage causes further exacerbation of inflammation, resulting in neutrophil activation, neutrophil-endothelial cell adhesion, and further elaboration of inflammatory cytokines. These inflammatory processes further contribute to vascular endothelial dysfunction. Endogenous modulators of homeostasis, such as protein C and anti-thrombin III (AT III), are consumed and their levels become deficient as the body attempts to return to a normal functional state. Under normal conditions, the endothelial surface proteins thrombomodulin and endothelial protein C receptor (EPCR), activate protein C and its modulating effects. In sepsis, the endothelial damage impairs this function of thrombomodulin and EPCR, thereby contributing to the loss of control. Left unopposed, the endothelial damage accumulates. This uncontrolled cascade of inflammation and coagulation fuels the progression of sepsis, resulting in hypoxia, widespread ischemia, organ dysfunction, and ultimately death for a large number of patients.

By the phrase "sepsis-related condition" is meant those known to precede, accompany, or follow sepsis including, but not limited to, disseminated intravascular coagulation (DIC), fibrin deposition, thrombosis, and lung injury, including acute lung injury (ALI), or acute respiratory distress syndrome (ARDS). A particular type of lung injury amenable to prevention or treatment by use of the invention is sepsis-induced acute lung injury. See Welty-Wolf, et al., Am. J. Respir. Crit. Care Med. 164:1988 (2001), for instance. Also encompassed are certain renal disorders accompanying sepsis such as acute tubular necrosis (ACN) and related conditions.

Acute lung injury (ALI) and acute respiratory distress syndrome (ARDS) are severe disorders that continue to receive significant attention. In particular, an important feature of ALI and ARDS, is local activation of extrinsic coagulation and inhibition of fibrinolysis. As the injury evolves, these perturbations have been reported to cause deposition of fibrin in the microvascular, interstitial, and alveolar spaces of the lung leading to capillary obliteration and hyaline membrane formation. Components of the extrinsic coagulation pathway (*e.g*. tissue factor (TF), thrombin, and fibrin) signal alterations in inflammatory cell traffic and increases in vascular permeability. Procoagulants and fibrin are also thought to promote other key events in the injury including complement activation, production of pro-inflammatory cytokines, inhibition of fibrinolysis, and remodeling of the injured lung. Without wishing to be bound to theory, it is believed that by reducing or blocking these initiating events (extrinsic coagulation) and downstream effects (pro-inflammatory events) in the lungs, disordered fibrin turnover can be reduced or blocked and the evolution of severe structural and functional injury can be reduced or averted during ALI/ARDS. Coagulation blockade can target the TF-Factor VIIa (TF-FVIIa) complex at several points, but the effects of these different strategies on inflammation and the development of lung injury are still being confirmed. The Examples show use of a chimerized monoclonal antibody against human TF (cH36) and its Fab fragment (cH36-Fab) in blocking initiation of coagulation in gram-negative sepsis and prevent acute lung injury.

The present disclosure is intended, in one embodiment, to prevent or treat sepsis and sepsis related conditions (such as DIC, ALI and ARDS) by reducing or blocking activity of a key component of the blood coagulation cascade (*i.e*., TF). Preferred humanized antibodies, chimeric antibodies, and fragments thereof specifically bind human TF typically to block at least one of FX or Factor IX binding g to the TF complex. Typically, such preferred antibodies and fragments also inhibit or block FX or FIX activation of that complex. Thus, the compositions and methods of the disclosure reduce or block unwanted activation of the blood coagulation cascade by decreasing or preventing activity of a key molecular component.

Such preferred antibodies are different from prior antibodies such as those provided by U.S. Pat. No. 6,274,142 ('"142") to O'Brien et al. For instance, the '142 patent reports TF neutralizing antibodies that cannot bind to Factor VII/VIIa or effect proteolysis of Factors IX or X. In contrast, preferred antibodies of the disclosure substantially reduce or block at least one of FX or FIX binding to the TF complex. See also PCT/USOll07501 (WO 01/70984).

As mentioned previously, unwanted coagulation activation is a prominent feature of certain inflammatory diseases, particularly those associated with autoimmunity. See Weinberg, et al., Arthritis Rheum. 34:996-1005 (1991); Kincaid-Smith, Kidney Int 7:242-253 (1975); Wakeheld, et al., J Clin Pathol 47:129-133 (1994); Schoph, et al., Arch Dermatol Res 285:305-309 (1993); Zeher, et al., Clin Immunol Immunopathol 71:149-155 (1994); More, et al., J Clin Pathol 46:703-708 (1993). Elevated TF levels have been reported to be associated with disease activity in systemic lupus erythematosus. See Segal, et al., J Rheumatol 27:2827-2832 (2000). It is thus an object of this invention to help control tissue factor-mediated coagulation, thereby reducing or in some instances blocking the inflammation, pathological tissue destruction and undesired remodeling that is a hallmark of many inflammatory diseases.

By the phrase "inflammatory disease" including plural forms is meant a pathological condition associated with an unwanted TF-mediated activation of the coagulation. Preferred inflammatory diseases are usually associated with a known or suspected autoimmune condition. Typically, such diseases are further associated with enhanced production of pro-inflammatory cytokines and/or chemokines as determined by standard methods. Examples of such cytokines include IL-1, TNFα, GM-CSF, M-CSF, IL-6, LIF, IL-15, IFNα, and IL12. Examples of such chemokines include IL-8, MIP-1α, MIP-1β, MCP-1, ENA-78, and RANTES. Typically, but not exclusively, one or more of neovascularization and extravascular fibrin deposition is associated with the inflammatory disease. More particular examples of inflammatory diseases according to the disclosure include arthritis, preferably rheumatoid arthritis (RA); glomerulonephritis, multiple sclerosis, psoriasis, Sjogren's syndrome and inflammatory bowel disease. Arthritis can be readily detected by one or a combination of features including presence of synovial inflammation, pannus formation, and cartilage destruction.

Preferred use of the disclosure will help reduce, prevent or alleviate symptoms of the inflammatory disease typically by decreasing TF-mediated coagulation activation. Such activation by TF is believed to provide many disadvantages including, but not limited to, supporting the production of inflammatory molecules, enhancing proinflammatory cell activity, increasing tissue destruction, increasing unwanted remodeling and boosting angiogenesis. The disclosure thus provides a new and fundamental means for addressing inflammatory disease by providing compositions and methods for specifically binding and inhibiting function of TF.

Practice of the disclosure will particularly help in the prevention and treatment of inflammatory autoimmune diseases. Recent work is in agreement with this inventive concept. See Marty, et al., J Clin Invest 107:631-640 (2001); Varisco, et al., Ann Rheum Dis 59:781-787 (2000); Busso, et al., Arthritis Rheum 48:651-659 (2003).

As discussed, the U.S. Pat. Nos. 5,986,065 and 6,555,319 to Wong, H. et al. and PCT/US98/04644 (WO 98/40408) disclose a variety of murine anti-TF antibodies and antigen binding fragments with good human TF binding characteristics. Such antibodies and fragments can be employed in accord with the present invention. Additionally, such antibodies and fragments can be used to treat experimentally induced sepsis, for instance, in a relevant rodent model. However, and as will be appreciated, such murine antibodies and fragments are not usually appropriate for use in other mammals such as primates and especially human subjects. Further suitable antibodies and fragments are disclosed by U.S. Patent Application Publication No. 20030082636; WO 03/037911 and WO 98/40408.

By the phrase "antigen binding fragment" is meant at least a part of an antibody that specifically binds antigen. An example of such a fragment includes an antibody V domain. Examples of a suitable V domain binding partner include a C domain and acceptable fragments thereof. Further suitable fragments include parts of the V domain having a combined molecular mass for the V domain of between from about 15 kilodaltons to about 40 kilodaltons, preferably between from about 20 kilodaltons to about 30 kilodaltons, more preferably about 25 kilodaltons as determined by a variety of standard methods including SDS polyacrylamide gel electrophoresis or size exclusion chromatography using appropriately sized marker fragments, mass spectroscopy or amino acid sequence analysis. Further specific antigen binding fragments include Fab, F(v), Fab', F(ab')₂ and certain single-chain constructs that include the antibody V domain.

Additionally suitable antigen binding fragments include at least part of an antigen binding V domains alone or in combination with a cognate constant (C) domain or fragment thereof ("cognate" is used to denote relationship between two components of the same immunoglobulin heavy (H) or light (L) chain). Typical C domain fragments have a molecular mass of between from about 5 kilodaltons to about 50 kilodaltons, more preferably between from about 10 kilodaltons to about 40 kilodaltons, as determined by a variety of standard methods including SDS polyacrylamide gel electrophoresis or size exclusion chromatography using appropriately sized marker fragments, mass spectroscopy or amino acid sequence analysis. Additionally suitable antigen binding fragments are disclosed below as well as in U.S. Pat. Nos. 5,986,065 and 6,555,319 to Wong, H. et al. and PCT/US98/04644 (WO 98/40408). See also U.S. Patent Application Publication No. 20030082636 and the following International Applications: WO 03/037911 and WO 98/40408.

As also mentioned, one approach to minimize any potential immunorejection by primate hosts such as human patients is to make a chimeric antibody. By the phrase "chimeric antibody" or related phrase including plural forms is meant antibodies as disclosed herein whose light and heavy chain genes have been constructed, typically by genetic engineering, from immunoglobulin gene segments belonging to different species, usually a primate and preferably a human. For example, the variable (V) domains of the genes from a mouse antibody such as H36 may be joined to human constant (C) domains, such as γ₁, γ₂, γ₃, or γ₄. A typical therapeutic chimeric antibody is thus a hybrid protein consisting of the V or antigen-binding domain from a mouse antibody and the C or effector domain from a human antibody, although other mammalian species may be used. A specifically preferred chimeric antibody for use with the invention is the anti-tissue factor antibody cH36 disclosed below in the Examples.

Suitable chimeric antibodies for use with the invention can be made by one or a combination of known strategies. As disclosed in the U.S. Pat. Nos. 5,986,065, 6,555,319 and PCT/US98/04644 (WO 98/40408), a highly useful murine anti-TF antibody can be readily obtained from a variety of sources including the American Type Culture Collection (ATCC, 10801 University Boulevard, Manassas, VA 20110). The antibody has been deposited as ATCC Accession No. HB-12255. Alternatively, suitable antibodies can be made *de novo* (as polyclonal or monoclonal as needed) in accord with procedures disclosed in the U.S. Pat. Nos. 5,986,065 and 6,555,319 for instance.

See also U.S. Patent Application Publication No. 20030082636 and the following International Applications: WO 03/037911 and WO 98/40408.

The antibody deposited with the ATCC H36 is referred to herein as H36. It is also referenced as H36.D2 and as H36.D2.B7. The antibody designated as H36 is the antibody produced by the mother clone, and H36.D2 is obtained from the primary clone, whereas H36.D2.B7 is obtained from the secondary clone. No differences were observed between the antibody produced by those three clones with respect to the antibody's ability to inhibit TF or other physical properties. In general usage, H36 is often used to indicate anti-TF antibody produced by any of these clones or related cell lines producing the antibody. The mouse-human chimeric version of H36 is referred to cH36 (and also as Sunol-cH36). See also the U.S. Pat. No. 5,986,065 and PCT/US98/04644 (WO 98/40408) for more specific information about the H36 antibody.

A preferred chimeric antibody combines the murine variable domain from a suitable antibody such as H36 and a human constant domain. The manipulation is usually achieved by using standard nucleic acid recombination techniques. A variety of types of such chimeric antibodies can be prepared, including e.g. by producing human variable domain chimeras, in which parts of the variable domains, especially conserved regions of the antigen-binding domain, are of human origin and only the hypervariable regions are of non-human origin. See S.L. Morrison, Science, 229:1202-1207 (1985); Oi et al., BioTechniques 4:214 (1986); Teng et al., Proc. Natl. Acad. Sci. U.SA., 80:7308-7312 (1983); Kozobor et al., Immunology Today 4:72-79 (1983); Olsson et al., Meth. Enzymol. 92:3-16 (1983); U.S. Pat. No. 5,986,065; and PCT/US98/04644 (WO 98/40408).

Nucleic acids encoding the H36 variable and constant regions have been disclosed in the U.S. Pat. No. 5,986,065; and PCT/US98/04644 (WO 98/40408), for example. See also U.S. Patent Application Publication No. 20030082636; WO 03/037911 and WO 98/40408.

In one embodiment, the anti-TF chimeric antibody will include a human light chain constant (C) domain *i.e*., Cκ, C_{λ} or a fragment thereof. Often, the humanized light chain fragment will have an amino acid length of between from about 80 to about 250 amino acids, preferably between from about 95 to about 235 amino acids, more preferably between from about 104 to about 225 amino acids. The size of the humanized light chain fragment can be determined by a variety of standard methods including SDS polyacrylamide gel electrophoresis or size exclusion chromatography using appropriately sized marker fragments, mass spectroscopy or amino acid sequence analysis.

Typically, the chimeric antibody for use in the present methods further includes a human heavy chain variable (V) domain having an amino acid length of between about 80 to about 650 amino acids, preferably between from about 95 to about 540 amino acids, more preferably about 102 to about 527 amino acids as determined *e.g*., by standard SDS polyacrylamide gel electrophoresis or size exclusion chromatography using appropriately sized marker fragments; mass spectroscopy or amino acid sequence analysis.

Nucleic acid sequence encoding suitable human light chain C and V domains have been reported. See *e.g.,* Kabat et al. in Sequences of Proteins of Immunological Interest Fifth Edition, U.S. Dept. of Health and Human Services, U.S. Government Printing Office (1991) NIH Publication No. 91-3242; and GenBank. See the National Center for Biotechnology Information (NCBI)- Genetic Sequence Data Bank (Genbank) at the National Library of Medicine, 38A, 8N05, Rockville Pike, Bethesda, MD 20894. See Benson, D.A. et al., Nucl. Acids. Res. 25:1 (1997) for a more specific description of Genbank.

Suitable recombinant techniques for use in making the chimeric antibodies and other antibodies and fragments as reported herein have been disclosed. See generally Sambrook et al. in Molecular Cloning: A Laboratory Manual (2d ed. 1989); and Ausubel et al., Current Protocols in Molecular Biology, John Wiley & Sons, New York, (1989).

For some applications in which a minimal immunoresponse against an anti-TF antibody is needed, it will be useful to prepare humanized antibodies. By the phrase "humanized" is meant an immunoglobulin that includes at least one human FR subset, preferably at least two or three of same, more preferably four human FR subsets, and one or more CDRs from a non-human source, usually rodent such as a rat or mouse immunoglobulin. Typically preferred humanized immunoglobulins of the disclosure will include two or more preferably three CDRs. Constant domains need not be present but are often useful in assisting function of humanized antibodies intended for *in vivo* use. Preferred constant domains, if present, are substantially identical to human immunoglobulin constant domains *i.e*., at least about 90% identical with regard to the amino acid sequence, preferably at least about 95% identical or greater. Accordingly, nearly all parts of the humanized immunoglobulin, with the possible exception of the CDRs, are preferably substantially identical to corresponding parts of naturally occurring human immunoglobulin sequences.

Methods for determining amino acid sequence identity are standard in the field and include visual inspection as well as computer-assisted approaches using BLAST and FASTA (available from the National Library of Medicine (USA) website). Preferred matching programs for most embodiments are available from website for the international ImMunoGeneTics (IMGT) database and a more preferred matching program for this embodiment is the program called Match which is available in the Kabat database. See Johnson G, Wu T. "Kabat database and its application: Future directions." Nucleic Acids Res, 29:205-206 (2001).

By the phrase "humanized antibody" is meant an antibody that includes a humanized light chain and a humanized heavy chain immunoglobulin. See S.L. Morrison, *supra;* Oi *et al., supra;* Teng *et al., supra;* Kozbor *et al., supra;* Olsson *et al., supra;* and other references cited previously. Accordingly, a "humanized antibody fragment" means a part of that antibody, preferably a part that binds antigen specifically.

The H36 or cH36 antibody can be humanized by one or a combination of approaches as described, for example, in U.S Pat: Nos. 5,766,886; 5,693,762; 5,985,279; 5,225,539; EP-A-0239400; 5,985,279 and 5,639,641, or as disclosed in the published U.S. application number 20030190705. See also E. Padlan Mol. Immunol. 28:489 (1991); Jones et al., Nature 321:522-525 (1986); Junghans *et al., supra;* and Roguska, et al. PNAS (USA) 91:969 (1994) for additional information on humanizing antibodies.

Particular humanized antibodies and fragments thereof:
are disclosed below in the Examples section.

Preferred chimeric antibodies, humanized antibodies, as well as fragments thereof that specifically bind human TF. By the term, "specific binding" or a similar term is meant a molecule disclosed herein which binds another molecule, thereby forming a specific binding pair. However, the molecule does not recognize or bind to other molecules as determined by, *e.g*., Western blotting ELISA, RIA, mobility shift assay, enzyme-immunoassay, competitive assays, saturation assays or other protein binding assays know in the art. See generally, Sambrook et al. in Molecular Cloning: A Laboratory Manual (2nd ed. 1989); and Ausubel *et al.,* supra; and Harlow and Lane in Antibodies: A Laboratory Manual (1988) Cold Spring Harbor, New York for examples of methods for detecting specific binding between molecules.

Especially suitable chimeric antibodies, humanized antibodies and fragments for use with the disclosure will feature at least one of: 1) a dissociation constant (K_{d}) for the TF of less than about 0.5 nM; and 2) an affinity constant (Kₐ) for the TF of less than about 10 x 10¹⁰ M⁻¹. Methods of preforming such assays are known in the field and include Enzyme-Linked Immuno-Sorbent Assay (ELISA), Enzyme ImmunoAssay (EIA) radioimmunoassay (RIA) and BIAcore analysis.

Additionally suitable antibodies will increase survival time in what is sometimes referred to herein as a "standard *in vivo* septic shock assay". Generally, such an assay involves administering gram-negative bacteria to monkeys to induce sepsis. See Taylor et al. J. Clin. Invest. 79:918 (1987). More specifically, a dose of *E. coli* (about 10¹⁰ CFU/kg) is freshly prepared and administered intravenously to a primate, *e.g*. baboon or rhesus monkey, in a 1-2 hour interval. A control group can receive a saline or PBS injection. The treatment group receives a bolus injection of at least 1 mg/kg antibody, preferably about 10 mg/kg prior to infusion of bacteria *e.g*., less than about 1 hour before. Control and treatment group monkeys are then monitored for about a week and checked for survival. See the Examples below for more specific information about the standard *in vivo* septic shock assay.

A preferred method of the disclosure employs a humanized antibody, chimeric antibody or fragment that increases monkey survival time (hours or days) by at least about 2-fold, preferably at least about 3 fold, more preferably at least about 5 fold to 10 fold or more as determined by the standard *in vivo* septic shock assay.

Additionally suitable antibodies for use with the disclosure can attenuate (reduce presence of at least one of interleukin-6 (IL-6) and interleukin-8 (IL-8) in the plasma of subject mammals after at least about 5 hours following administration of the antibody. Methods for detecting IL-6 and IL-8 and quantifying same from plasma are known and include immunological approaches such as RIA, EIA, ELISA and the like.

In one approach, sepsis is induced in a suitable primate such as a monkey and preferably a baboon along lines previously mentioned. Prior to inoculation of control baboon (with about 10¹⁰ CFU/kg *E*. *coli* or saline), the treatment baboons receive a bolus injection of at least 1 mg/kg antibody, preferably about 10 mg/kg prior to infusion of bacteria e.g., less than about 1 hour before injection of the inoculum. Baboon survival and IL-6 and IL-8 levels in plasma are monitored by standard procedures.

In another approach, a sepsis-like condition is induced in a suitable primate, such as baboon, using a model described as a "primed sepsis model" (see Welty-Wolf, K. et al. Am. J. Respir. Crit. Care Med. 164:1988 (2001) and described in further detail in Example 4).

Additionally preferred humanized antibodies, chimeric antibodies and fragments thereof exhibit a blood clotting time of between from about 50 to about 350 seconds as determined by a standard prothrombin (PT) time assay, particularly after about 5 minutes administration of the antibody or fragment to the mammal. An especially useful PT assay has been disclosed in the U.S. Pat. Nos. 5,986,065, 6,555,319 and PCT/US98/04644 (WO 98/40408), for example.

Further preferred antibodies for use in accord with the present disclosure inhibit platelet deposition by at least about 50% as determined by a standard platelet: deposition assay. Methods for performing the assay have been disclosed, *e.g*., in the pending U.S. Pat. Application 10/310,113 (U.S. Publication Number 2003-0176664).

Still further preferred antibodies inhibit collagen-induced arthritis in an experimental mouse model. See Example 5.

"Antibody", "antibody for use with the invention" and like phrases refer to whole immunoglobulin as well as immunologically active fragments which bind a desired antigen. The immunoglobulins and immunogically active (antigen-binding) fragments thereof include an epitope-binding site (*i.e*., a site or epitope capable of being specifically bound by an antibody recognizing antigen). Exemplary antibody fragments include, for example, Fab, F(v), Fab', F(ab')₂ fragments, "half molecules" derived by reducing the disulfide bonds of immunoglobulins, single chain immunoglobulins, or other suitable antigen binding fragments (see *e.g*., Bird et al., Science, 242:423-426 (1988); Huston et al., PNAS, (USA), 85:5879 (1988); Webber et al., Mol. Immunol., 32:249 (1995)).

Particular chimeric or humanized antibodies, of the present disclosure can be polyclonal or monoclonal, as needed, and may have, without limitation, an IgG1, IgG2, IgG3 or IgG4 isotype or IgA, IgD, IgE, IgM. An especially preferred antibody for use with the invention has or can be manipulated to have an IgG1 (called "hOAT") or IgG4 (called "hFAT") isotype. Such antibodies can be polyclonal or monoclonal as needed to achieve the objectives of a particular invention embodiment. In some instances, single-chain antibodies such as a humanized single-chain will be preferred.

Practice of the invention is applicable to a wide spectrum of mammals. Preferably, the mammal is a primate such as a monkey, chimpanzee, or a baboon. More preferably, the primate is a human patient in need of methods disclosed herein i.e., one that has or is suspected of having sepsis or a sepsis-related condition or an inflammatory disorder or disease.

As discussed above, antibodies of the disclosure and suitable fragments thereof can be administered to a mammal, preferably a primate such as a human, to prevent or reduce sepsis and related complications. According to one embodiment, such antibodies are used with one or more pharmaceutically acceptable non-toxic carriers such as sterile water or saline, glycols such as polyethylene glycol, oils of vegetable origin, and the like. In particular, biocompatible, biodegradable lactide polymer, lactide glycolide copolymer or polyoxyethylene, polyoxypropylene copolymers may be useful excipients. Other potentially useful administration systems include ethylene vinyl acetate copolymer particles, osmotic pumps, and implantable infusion systems and liposomes. If needed, one or more suitable antibodies or fragments will be in the form of a solution or suspension (or a lyophilized form that can be reconstituted to a solution or suspension), and will preferably include approximately 0.01% to 10% (w/w) of the antibody of the present invention, preferably approximately 0.01 % to 5% (w/w) of the antibody.

As discussed, the antibody or fragment can be administered according to the disclosure as the sole active agent or in combination with other known anti-sepsis therapies. For instance, such antibodies or fragments can be administered to a human patient before, during, or after intervention with one or more appropriate antibotics, typically a broad spectrum intravenous antibiotic therapy. Preferred antibiotics include those known to have broad spectrum anti-microbial activity, covering gram-positive, gram-negative, and anaerobic bacteria. Thus in one embodiment, such antibiotics are given during or after administration of the antibodies and fragments disclosed herein, preferably parenterally in doses adequate to achieve bactericidal serum levels. After stabilizing the patient, various recognized supportive therapies can be used to assist recovery such as administration of oxygen, intravenous fluids, and medications that increase blood pressure. Dialysis may be necessary in the event of kidney failure, and mechanical ventilation is often required if respiratory failure occurs.

Persons "at risk" for developing sepsis and related conditions include, but are not limited to, the very old and very young individuals. Also at risk are those with challenged immune systems such as patients infected with a DNA or RNA virus such as HIV or herpes. Nearly any bacterial organism can cause sepsis. Certain fungi and (rarely) viruses may faciliate sepsis and related conditions. Generally, toxins released by the bacteria or fungus may cause direct organ (*e.g*., lung, kidney) or tissue damage, and may lead to low blood pressure and/or poor organ function. These toxins also produce a vigorous inflammatory response from the body which contributes to septic shock.

Additional risk factors include underlying illnesses, such as diabetes; hematologic cancers (lymphoma or leukemia); and other malignancies and diseases of the genitourinary system, liver or biliary system, and intestinal system. Other risk factors are recent infection, prolonged antibiotic therapy, and having been exposed to a recent invasive surgical or medical procedure. Symptoms of sepsis and related conditions are known in the field and include, but are not limited to, fever, chills, lightheadedness, shortness of breath, palpitations, cool and/or pale extremities, fever, agitation, lethargy, and confusion.

Success of the disclosure in the prevention or treatment of sepsis and related conditions can be evaluated by the caregiver using one or a combination of approaches. Typically, a reduction or elimination of one or more of the foregoing symptoms can be taken as indicative that the sepsis or related condition has been addressed. Preferably, patients receiving such treatment will survive at least about 2 fold longer than patients who do not receive such intervention.

As discussed, the disclosure provides a method for reducing cytokine production in a mammal *e.g*., by administering to the mammal a therapeutically effective amount of at least one humanized antibody, chimeric antibody, or fragment thereof that binds specifically to tissue factor (TF) to form a complex. Preferably, Factor X or Factor IX binding to the complex is inhibited and the administration is sufficient to reduce the cytokine production in the mammal. Suitable humanized antibodies, chimeric antibodies and fragments thereof are disclosed herein. Acceptable methods for monitoring cytokine production in a mammal are known and include specific methods outlined in the Examples section.

Therapeutic antibodies and fragments in accord with the invention can be used in parenteral or intravenous administration, particularly in the form of liquid solutions. Such compositions may be conveniently administered in unit dose and may be prepared in accordance with methods known in the pharmaceutical art. See Remington's Pharmaceutical Sciences, (Mack Publishing Co., Easton PA, (1980)). By the term "unit dose" is meant a therapeutic composition of the present disclosure employed in a physically discrete unit suitable as unitary dosages for a primate such as a human, each unit containing a pre-determined quantity of active material calculated to produce the desired therapeutic effect in association with the required diluent or carrier. The unit dose will depend on a variety of factors including the type and severity of sepsis to be treated, general health of the individual, medical history, and the like. Precise amounts of the antibody to be administered typically will be guided by judgment of the practitioner, however, the unit dose will generally depend on the route of administration and be in the range of 10 ng/kg body weight to 50 mg/kg body weight per day, more typically in the range of 100 ng/kg body weight to about 10 mg/kg body weight per day. Suitable regimens for initial administration in booster shots are also variable but are typified by an initial administration followed by repeated doses at one or more hour intervals by a subsequent injection or other administration. Alternatively, continuous or intermittent intravenous infusions may be made sufficient to maintain concentrations of at least from about 10 nanomolar to 10 micromolar of the antibody (or suitable fragment) in the blood.

Antibodies and fragments for use with the invention are preferably substantially pure when used in the disclosed methods and assays. References to an antibody being "substantially pure" mean an antibody or protein that has been separated from components which naturally accompany it. For example, by using standard immunoaffinity or protein A affinity purification techniques, an antibody of the invention can be purified from hybridoma or cell culture medium by using native TF as an antigen or protein A resin. Similarly, native TF can be obtained in substantially pure form by using an antibody of the invention with standard immunoaffinity purification techniques. Particularly, an antibody or protein is substantially pure when at least 50% of the total protein (weight % of total protein in a given sample) is an antibody or protein of the invention. Preferably the antibody or protein is at least 60 weight % of the total protein, more preferably at least 75 weight %, even more preferably at least 90 weight %, and most preferably at least 98 weight % of the total material. Purity can be readily assayed by known methods such as SDS polyacrylamide gel electrophoresis (PAGE), column chromatography (*e.g*., affinity chromatography, size exclusion chromatography), mass spectroscopy or HPLC analysis. Preferably, the antibodies and fragments will be used in a sterile format.

The molecular weight of the antibodies of the invention will vary depending on several factors such as the intended use and whether the antibody includes a conjugated or recombinantly fused toxin, pharmaceutical, radioisotope or detectable label or the like. Also the molecular weight will vary depending on nature and extent of post-translational modifications if any (such as glycosylation) to the antibody. The modifications are a function of the host used for expression with *E. coli* producing non-glycosylated antibodies and eucaryotic hosts, such as mammalian cells or plants, producing glycosylated antibodies. In general, an antibody of the invention will have a molecular weight of between approximately 20 to 150kDa. Such molecular weights can be readily determined by molecular sizing methods such as SDS-PAGE followed by protein staining or Western blot analysis.

It will be apparent that the foregoing method for making the humanized cH36 antibody can be readily adapted to make other humanized antibodies and antigen binding fragments according to the invention.

### A. Preparation Of Humanized Anti-Tissue Factor Binding Antibody

Preparation and use of a humanized anti-tissue factor binding antibody is described. See also Examples 1-3 below.

Briefly, preferred antibodies bind human tissue factor to form a binding complex. The tissue factor may be naturally occurring or recombinant human (rhTF). Preferably, factor X or factor IX binding to the complex is inhibited. In a preferred disclosure embodiment, the humanized antibody has an apparent affinity constant (K_{A}, M⁻¹) for the hTF of less than about 1 nM, preferably less than about 0.5 nM, more preferably between from about 0.01 nM to about 0.4 nM. See Examples 1-3, below for more information about determining affinity constants for the humanized antibodies. By "specific binding" is meant that the humanized antibodies form a detectable binding complex with the TF (or rhTF) and no other antigen as determined by standard immunological techniques such as RIA, Western blot or ELISA.

More preferred humanized anti-TF binding antibodies made in accord with this disclosure exhibit an apparent affinity constant (K_{A}, M⁻¹) for native human TF of at least about 1 x 10⁸ M⁻¹ as determined by surface plasmon analysis (particularly, BIACore analysis in accordance with the procedures of Example 3 which follows), more preferably at least about 5 x 10⁸ M⁻¹ as determined by surface plasmon analysis, still more preferably an apparent affinity constant (K_{A}, M⁻¹) for native human TF of at least about 3 x 10⁹ M⁻¹ as determined by surface plasmon resonance analysis. Such substantial binding affinity of antibodies of the disclosure contrast sharply from much lower binding affinities of previously reported antibodies.

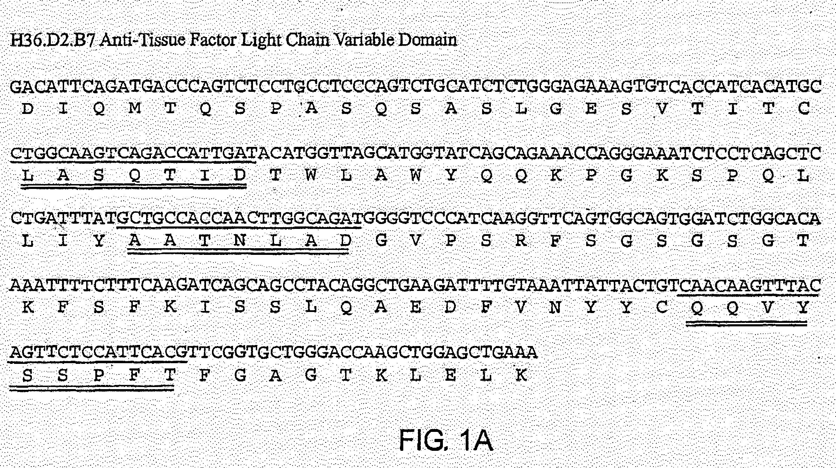

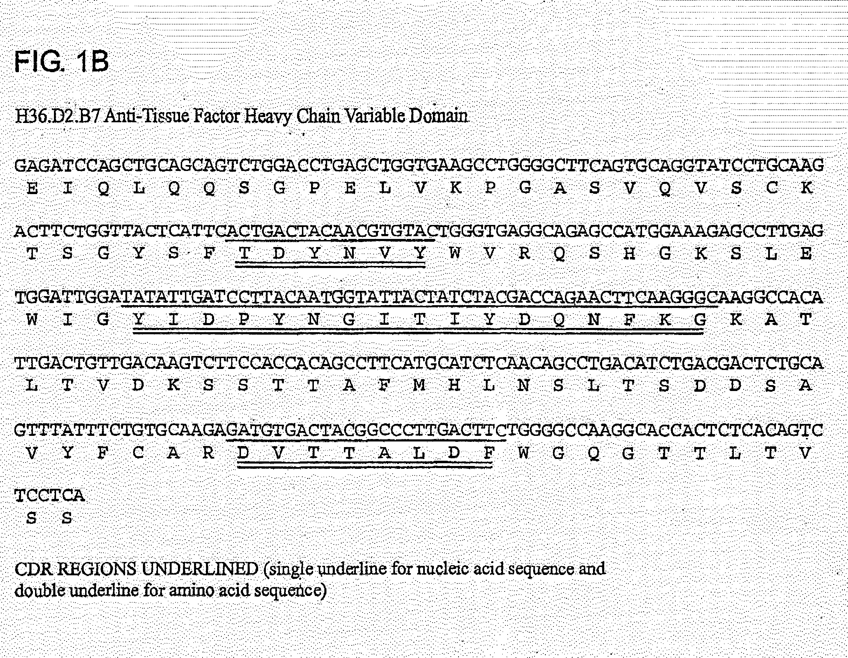

The nucleic acid (SEQ ID NOS. 1 and 3) and amino acid (SEQ ID NOS. 2 and 4) sequences of a particular tissue factor binding antibody that has been humanized by the present methods i.e. H36.D2.B7. See Figures 1A and 1B of the drawings and the PCT application WO 98/40408, for instance. SEQ ID NOS. 1 and 2 are the nucleic acid and amino acid respectively of the light chain variable domain, and SEQ ID NOS. 3 and 4 are the nucleic acid and amino acid respectively of the heavy chain variable domain, with hypervariable regions (CDRs or Complementarity Determining Regions) underlined in all of those sequences.

Additional tissue factor binding humanized antibodies of the invention will have substantial amino acid sequence identity to either one or both of the light chain or heavy sequences shown in Figures 1A and 1B. More particularly, such antibodies include those that have at least about 70 percent homology (amino acid sequence identity) to SEQ ID NOS. 2 and/or 4, more preferably about 80 percent or more homology to SEQ ID NOS. 2 and/or 4, still more preferably about 85, 90 or 95 percent or more homology to SEQ ID NOS. 2 and/or 4.

More particular tissue factor binding humanized antibodies of the disclosure will have high amino acid sequence identity to hypervariable regions (shown with double underlining in Figures 1A and 1B) of SEQ ID NOS. 2 and 4). Specific antibodies will have one, two or three hypervariable regions of a light chain variable domain that has high sequence identity (at least 90% or 95% amino acid sequence identity) to or be the same as one, two or three of the corresponding hypervariable regions of the light chain variable domain of H36.D2.B7 (those hypervariable regions shown with underlining in Figure 1A and are the following:
1) LASQTID (SEQ ID NO.5);
2) AATNLAD (SEQ ID NO.6); and
3) QQVYSSPFT (SEQ ID NO.7).

Additionally specific antibodies that have been humanized by the methods described herein and bind tissue factor will have one, two or three hypervariable regions of a heavy chain variable domain that have high sequence identity (at least 90% or 95% amino acid sequence identity) to or be the same as one, two or three of the corresponding hypervariable regions of the heavy chain variable domain of H36.D2.B7 (those hypervariable regions shown with underlining in Figure 1B and are the following:
1) TDYNVY (SEQ ID NO. 8);
2) YIDPYNGITIYDQNFKG (SEQ ID NO. 9); and
3) DVTTALDF (SEQ ID NO. 10).

Certain nucleic acids encoding some or all of the antibodies or fragments disclosed herein will preferably have a length sufficient (preferably at least about 100, 200 or 250 base pairs) to bind to the sequence of SEQ ID NO. 1 and/or SEQ ID NO. 3 under the following moderately stringent conditions (referred to herein as "normal stringency" conditions): use of a hybridization buffer comprising 20% formamide in 0.9M saline/0.12M sodium citrate (6xSSC) buffer at a temperature of 37°C and remaining bound when subject to washing once with that 2xSSC buffer at 37°C.

More specifically, certain of the nucleic acids (preferably at least about 100, 200 or 250 base pairs) will bind to the sequence of SEQ ID NO. 1 and/or SEQ ID NO.3 under the following highly stringent conditions (referred to herein as "high stringency" conditions): use of a hybridization buffer comprising 20% formamide in 0.9M saline/0.12M sodium citrate (6xSSC) buffer at a temperature of 42°C and remaining bound when subject to washing twice with that 1xSSC buffer at 42°C.

Suitable nucleic acids preferably comprise at least 20 base pairs, more preferably at least about 50 base pairs, and still more preferably a nucleic acid of the invention comprises at least about 100, 200, 250 or 300 base pairs.

Generally preferred nucleic acids of the disclosure will express an antibody that exhibits the preferred binding affinities and other properties as disclosed herein. See also the U.S. Pat. No. 5,986,065 and PCT/US98/04644 (WO 98/40408) for more information.

Other appropriate nucleic acids will have substantial sequence identity to either one or both of the light chain or heavy sequences shown in Figures 1A and 1B. More particularly, preferred nucleic acids will comprise a sequence that has at least about 70 percent homology (nucleotide sequence identity) to SEQ ID NOS. 1 and/or 3, more preferably about 80 percent or more homology to SEQ ID NOS. 1 and/or 3, still more preferably about 85, 90 or 95 percent or more homology to SEQ ID NOS. 1 and/or 3.

Additionally specific nucleic acid sequences will have high sequence identity to hypervariable regions (shown with underlining in Figures 1A and 1B) of SEQ ID NOS. 1 and 3). Such nucleic acids include those that code for an antibody light chain variable domain and have one, two or three sequences that code for hypervariable regions and have high sequence identity (at least 90% or 95% nucleotide sequence identity) to or be the same as one, two or three of the sequences coding for corresponding hypervariable regions of H36.D2.B7 (those hypervariable regions shown with underlining in Figure 1A and are the following:
1) CTGGCAAGTCAGACCATTGAT (SEQ ID NO: 11);
2) GCTGCCACCAACTTGGCAGAT (SEQ ID NO: 12); and
3) CAACAAGTTTACAGTTCTCCATTCACGT (SEQ ID NO: 13).

More specific nucleic acids also code for an antibody heavy chain variable domain and have one, two or three sequences that code for hypervariable regions and have high sequence identity (at least 90% or 95% sequence identity) to or be the same as one, two or three of the sequences coding for corresponding hypervariable regions of H36.D2.B7 (those hypervariable regions shown with underlining in Figure 1B and are the following:
1) ACTGACTACAACGTGTAC (SEQ ID NO. 14);
2) and
3) GATGTGACTACGGCCCTTGACTTC (SEQ ID NO. 16).

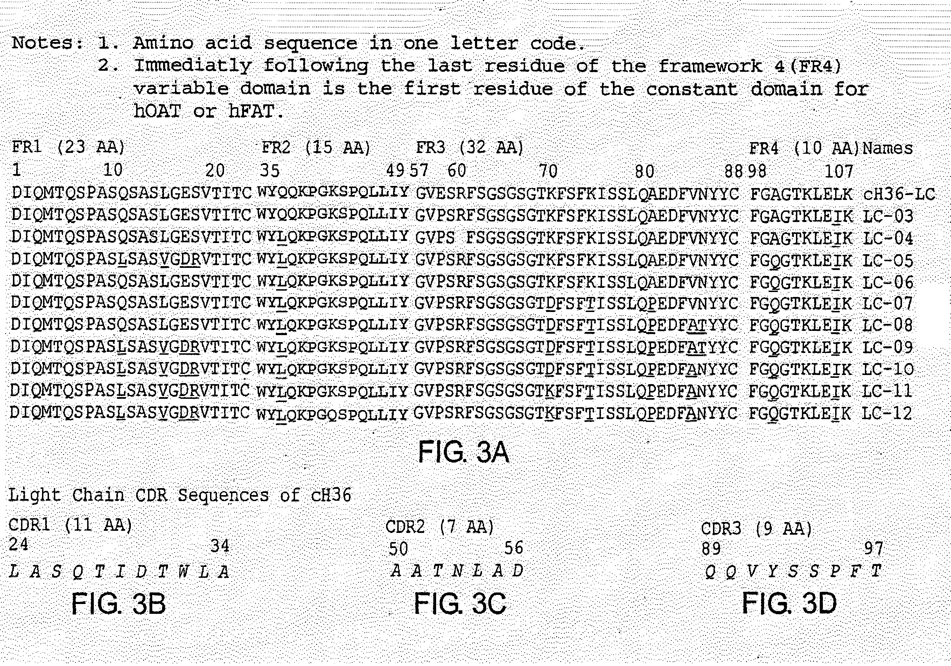

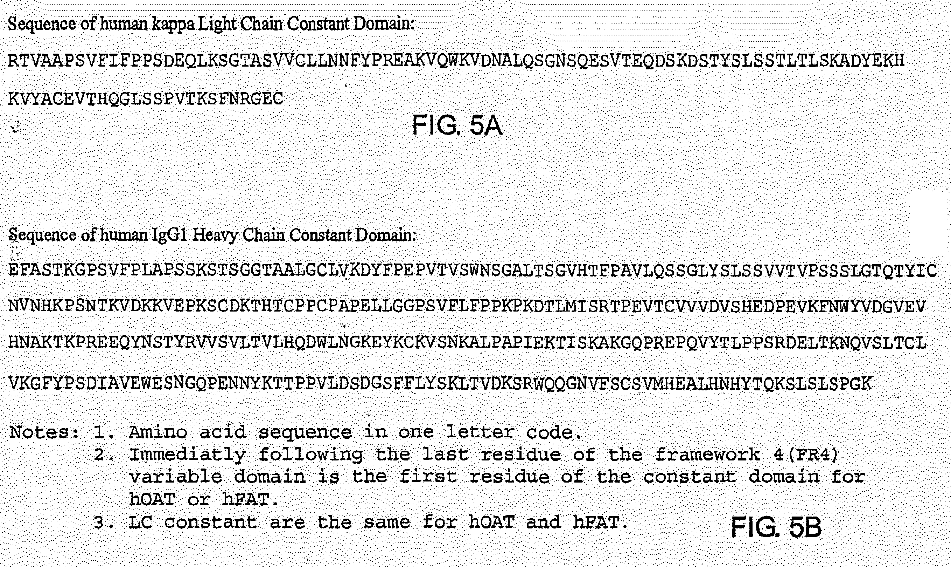

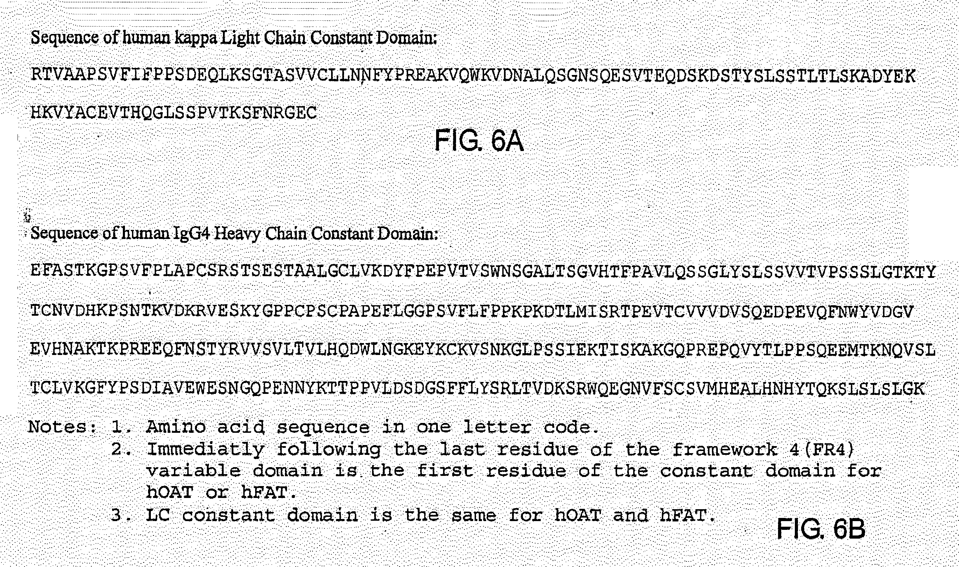

More specific humanized antibodies for use with the methods of this disclosure that bind TF are those in which each of framework regions (FRs) 1, 2, 3 and 4 has at least about 90% amino acid sequence identity, preferably at least about 95% or greater identity to the light chain FR sequences shown in Figure 3A, preferably, the sequence shown as "LC-09" in Figure 3A. Additionally specific humanized antibodies include a light chain constant domain having at least about 90% amino acid sequence identity, preferably at least about 95% sequence Identity or greater to the sequence shown in Figure 5A or Figure 6A.

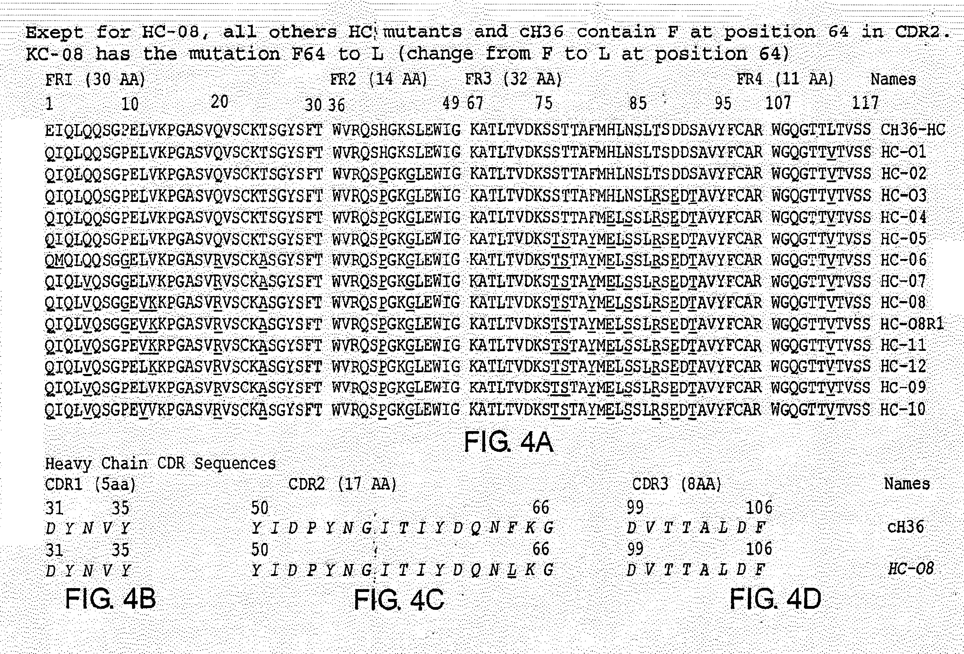

Further specific humanized antibodies are those in which each of framework regions (FRs) 1, 2, 3 and 4 has at least about 90% amino acid sequence identity, preferably about 95% identity or greater to the heavy chain sequences shown in Figure 4A, preferably, the sequence shown as "HC-08" in Figure 4A. Additional humanized antibodies have a heavy chain constant domain with at least about 90% amino acid sequence identity, preferably at least about 95% identity or greater, to sequence shown in Figure 5B or Figure 6B.

In certain embodiments, the humanized antibody will have an IgG1 (hOAT) or IgG4 (hFAT) isotype as disclosed in the published U.S. application number 20030190705.

Also provided by the present disclosure are functional fragments of the humanized antibodies disclosed herein. Examples of such fragments include, but are not limited to, those that bind TF with an affinity constant (Kd) of less than about 1 nM, preferably less than about 0.5 nM, more preferably between from about 0.01 nM to about 0.4 nM. Specifically preferred are antigen binding Fab, Fab', and F(ab)₂ fragments.

As discussed, the disclosure features humanized antibodies that include at least one murine complementarity determining region (CDR), e.g., CDR1, CDR2, CDR3. In one embodiment, the antibodies bind specifically to human tissue factor (TF) to form a complex. Typically, the factor X or factor IX binding to TF or TF-FVIIa and activation by TF-FVIIa thereto is inhibited. As mentioned above, preferred CDRs (light and heavy chain) are from a rodent source, typically the mouse.

In one embodiment of the humanized antibodies of the disclosure, the antibodies further include at least one human framework region (FR) subset. Preferably, all the FRs (light and heavy chains) are human.

In a more particular embodiment, the first CDR (CDR1) of the heavy chain hypervariable region that binds human TF is at least 90% identical to the CDR1 amino acid sequence shown in Figure 4B preferably at least about 95% identical or greater to that sequence. Typically, the second CDR (CDR2) of the heavy chain hypervariable region is at least 90% identical to the CDR2 amino acid sequence shown in Figure 4C, preferably at least about 95% identical or greater. Preferably also, the third CDR (CDR3) of the heavy chain hypervariable region is at least 90% identical to the CDR3 sequence shown in Figure 4D, more preferably about 95% identical or greater to that sequence.

In another disclosure embodiment, the first CDR (CDR1) of the light chain hypervariable region that binds human TF is at least 90% identical to the CDR1 amino acid sequence shown In Figure 3B, preferably at least about 95% identical or greater. Typically, the second CDR (CDR2) of the light chain hypervariable region is at least 90% identical to the CDR2 amino acid sequence shown in Figure 3C preferably about 95% identical or greater. Preferably, the third CDR (CDR3) of the light chain hypervariable region is at least 90% identical to the CDR3 amino acid sequence shown in Figure 3D, more preferably about 95% identical or greater to that sequence.

Additional humanized antibodies suitable for use with the present methods include a first framework region (FR1) of the heavy chain hypervariable region that binds human TF which FR1 is at least 90% identical to the amino acid sequence shown in Figure 4A as "FR1 HC-08", preferably about 95% identical or greater to that sequence. In one embodiment, the FR1 comprises at least one of the following amino acid changes: E1 to Q; Q5 to V; P9 to G; L11 to V; V12 to K; Q19 to R; and T24 to A. Preferably, the FR1 includes two, three, four, five, or six of those changes with all of those amino acid changes being preferred for many applications.

Further humanized antibodies suitably bind human TF and include a second framework region (FR2) of the heavy chain hypervariable region which FR2 is at least 90% identical to the sequence shown in Figure 4A as "FR2 HC-08", preferably about 95% identical or greater to that sequence. In one embodiment, the FR2 at least one of the following amino acid changes: H41 to P and S44 to G. A preferred FR2 includes both of those amino acid changes.

The invention also features use of humanized antibodies that bind human TF in which a third framework region (FR3) of the heavy chain hypervariable region is at least 90% identical to the sequence shown in Figure 4A as "FR3 HC-08", preferably about 95% identical or greater to that sequence. In one embodiment, the FR3 includes at least one of the following amino acid changes: S76 to T; T77 to S; F80 to Y; H82 to E; N84 to S; T87 to R; D89 to E and S91 to T. A preferred FR3 includes two, three, four, five or six of those amino acid changes with all seven of those amino acid changes being generally preferred.

Also featured is use of humanized antibodies that suitably bind human TF and in which the fourth framework region (FR4) of the heavy chain hypervariable region is at least 90% identical to the amino acid sequence shown in Figure 4A as "FR4 HC-08", preferably at least about 95% identical or greater to that sequence. Preferably, the FR4 includes the following amino acid change: L113 to V.

Additional humanized antibodies bind human TF and also feature a first framework region (FR1) of the light chain hypervariable region which is at least about 90% identical to the amino acid sequence shown in Figure 3A as "FR1 LC-09", preferably at least about 95% identical or greater to that sequence. In one embodiment, the FR1 comprises at least one of the following amino acid changes: Q11 to L; L15 to V; E17 to D; and S18 to R. A preferred FR1 includes two or three of such amino acid changes with all four amino acid changes being generally preferred.

The present invention also features use of humanized antibodies that bind human TF and in which a second framework region (FR2) of the light chain hypervariable region is at least about 90% identical to the amino acid sequence shown in Figure 3A as "FR2 LC-09", preferably at least about 95% identical or greater to that sequence. A preferred FR2 has the following amino acid change: Q37 to L.

Also encompassed by the invention is use of particular humanized antibodies that bind human TF in which a third framework region (FR3) of the light chain hypervariable region is at least about 90% identical to the amino acid sequence shown in Figure 3A as "FR3 LC-09", preferably at least about 95% identical or greater to that sequence. In one embodiment, the FR3 has at least one of the following amino acid changes: K70 to D, K74 to T, A80 to P, V84 to A, and N85 to T. Preferably, the FR3 has two, three, or four of such amino acid changes with all five of the changes being generally preferred.

Additional humanized antibodies appropriate for use with the methods disclosed herein bind TF and include a fourth framework region (FR4) of the light chain hypervariable region which FR4 is at least about 90% identical to the sequence shown in Figure 3A as "FR4 LC-09", preferably at least about 95% identical or greater to that sequence. In one embodiment, the FR4 includes at least one and preferably all of the following amino acid changes: A100 to Q and L106 to I.

The invention also features a human TF binding fragment of the foregoing humanized antibodies. Examples of such fragments include Fab, Fab', and F(ab)₂. See the published U.S. application number 20030190705 and references cited therein for additionally preferred humanized anti-TF antibodies made in accord with this disclosure.

The following three nucleic acid vectors pSUN36 (humanized anti-TF antibody Ig G1-HC expression vector), pSUN37 (humanized anti-TF antibody Ig G4-HC expression vector), and pSUN38 (humanized anti-TF antibody LC expression vector) have been deposited pursuant to the Budapest Treaty with the American Type Culture Collection (ATCC) at 10801 University Boulevard, Manassas VA 20110-2209. The vectors were assigned the following Accession Numbers: PTA-3727 (pSUN36); PTA-3728 (pSUN37); and PTA-3729 (pSUN38).

Suitable expression and purification strategies for making and using the humanized anti-TF antibodies of this disclosure have been disclosed in the published U.S. application number 20030190705, for instance.

As discussed, the disclosure also provides useful kits for performing one or more of the methods provided herein. In one embodiment, the kit includes at least one humanized antibody, chimeric antibody, or fragment thereof that binds specifically to human tissue factor (TF) to form a complex, wherein factor X or factor IX binding to the complex is inhibited. Typically also, the humanized antibody, chimeric antibody, or fragment thereof is provided in a pharmaceutically acceptable vehicle such as saline, water or buffer. The kit may include a pharmaceutically acceptable vehicle for dissolving the humanized antibody, chimeric antibody or fragment prior to use.

The following non-limiting examples are illustrative of the invention. In the following examples and elsewhere the method of the disclosure is applied to the humanization of the murine anti-tissue factor antibody H36. See the U.S Patent Nos. 5,986,065 and 6,555,319; U.S. Patent Application Publication No. 20030082636; WO 03/037911 and WO 98/40448, and the published U.S. patent application number 20030190705 and references cited therein as well as the discussion above. Sometimes, the Fab fragment of H36 or cH36 will be referred to as "H36-Fab" and "cH36-Fab", respectively, for the sake of convenience.

### EXAMPLE 1 - Humanization of Anti-Tissue Factor Antibody

The description of how to make and use a particular murine antibody called H36.D2 (sometimes also called H36 as discussed above) is described in U.S. Pat. Nos. 5,986,065 and 6,555,319. The present example shows how to make and use a humanized version of that antibody. A humanized H36 antibody has a variety of uses including helping to minimize potential for human anti-mouse antibody (HAMA) immunological responses. These and other undesired responses pose problems for use of the H36 antibody in human therapeutic applications.

### A. Preparation of chimeric anti-tissue factor antibody (cH36)

The H36 antibody described previously is an IgG2a murine antibody. H36 was first converted to a mouse-human chimeric antibody for clinical development. To do this, the heavy and light chain genes for H36 were cloned (see U.S. Patent No. 5,986,065). The heavy chain variable domain was fused to a human IgG4 constant (Fc) domain and the light chain variable domain was fused to a human kappa light chain constant domain. The resulting IgG4κ chimeric antibody was designated cH36 (and is also referred to as Sunol-cH36). For multiple uses of H36 or cH36 in patients with chronic diseases, a fully humanized cH36 is preferred so that it will decease or eliminate any human anti-chimeric antibody (HACA) immunological response. The humanization of cH36 is described below.

### B. Humanization Strategy for cH36 Antibody

Humanization of the chimeric anti-tissue factor antibody cH36 was achieved by using a "FR best-fit" method. This method takes full advantage of the fact that a great number of human IgGs with known amino acid sequences or sequences of human IgG fragments are available in the public database. The sequences of the individual framework regions of the mouse heavy and light variable domains in cH36 are compared with the sequences respective heavy or light chain variable domains or human frameworks (or fragments thereof) in the Kabat database (see *e.g*., Kabat et al. in Sequences of Proteins of Immunological Interest Fifth Edition, U.S. Dept. of Health and Human Services, U.S. Government Printing Office (1991) NIH Publication No. 91-3242 or http://immuno.bme.nwu.edu). The following criteria were used to select the desired human IgG framework region subsets for humanization: (1) The number of mismatched amino acids was kept as low as possible. (2) Amino acids inside the "vernier" zone (amino acids in this zone may adjust CDR structure and fine-tune the fit to antigen, see Foote, J. and Winter, G., J. of Mol. Bio. 224(2):487-499 [1992]) were left unchanged. (3) Conservative amino acid substitutions were favored when evaluating similar candidates. The matching program used for this comparison can be found in Kabat database. See Johnson G, Wu T. "Kabat database and its application: Future directions." Nucleic Acids Res. 29:205-206 (2001). The program finds and aligns regions of homologies between the mouse sequences and human sequences in the Kabat's database. By using this unique FR best-fit method, it is anticipated that the humanized LC or HC variable domains of the target IgG may have all the four FRs derived from as few as one human IgG molecule or to as many as four different human IgG molecules.

### B(i). Selection of Human IgG Kappa Light Chain Variable Domain Framework Regions

The amino acid sequence in each of the framework regions of cH36 LC was compared with the amino acid sequence in the FRs in human IgG kappa light chain variable domain in Kabat Database. The best-fit FR was selected based on the three criteria described above.

The amino acid sequence of human IgG kappa light chain variable domain with a Kabat Database ID No. 005191 was selected for humanization of cH36 LC FR1. The amino acid sequence of human IgG kappa light chain variable domain with a Kabat Database ID No. 019308 was selected for humanization of cH36 LC FR2. The following mutations were made in cH36 LC FR1 to match the amino acid sequence of a human IgG kappa light chain variable domain with a Kabat Database ID No. 005191: Q11 → L, L15 → V, E17 → D, S18 → R. One mutation Q37 → L was made cH36 LC FR2 to match the amino acid sequence of a human IgG kappa light chain variable domain with a Kabat Database ID No. 019308 (see Table 1A for sequence information).

The amino acid sequence of a human IgG kappa light chain variable domain with a Kabat Database ID No. 038233 was selected for humanization of cH36 LC FR3. The amino acid sequence of a human IgG kappa light chain variable domain with a Kabat Database ID No. 004733 was selected for humanization of cH36 LC FR4. The following mutations were made in cH36 LC FR3 to match the amino acid sequence of a human IgG kappa light chain variable region with a Kabat Database ID No. 038233: K70 → D, K74 → T, A80 → P, V84 → A, N85 → T. Two mutations A100 → Q and L106 → I were made cH36 LC FR4 to match the amino acid sequence of a human IgG kappa light chain variable domain with a Kabat Database ID No. 004733 (see Table 1B for sequence information).

### B(ii). Selection of Human IgG Heavy Chain Variable Domain Framework Regions

The amino acid sequence in each of the framework regions of cH36 HC was compared with the amino acid sequence in the FRs in human IgG heavy chain variable domain in Kabat Database. The best-fit FR was selected based on the three criteria described above.

The amino acid sequence of a human IgG heavy chain variable domain with a Kabat Database ID No. 000042 was selected for humanization of cH36 HC FR1. The amino acid sequence of a human IgG heavy chain variable domain with a Kabat Database ID No. 023960 was selected for humanization of cH36 HC FR2. The following mutations were made in cH36 HC FR1 to match the amino acid sequence of a human IgG heavy chain variable domain with a Kabat Database ID No. 000042: E1 → Q, Q5 → V, P9 → G, L11 → V, V12 → K, Q19 → R, T24 → A. Two mutations H41 → P and S44 → G were made cH36 HC FR2 to match the amino acid sequence of a human IgG heavy chain variable domain with a Kabat Database ID No. 023960 (see Table 2A for sequence information).

The amino acid sequence of a human IgG heavy chain variable domain with a Kabat Database ID No. 037010 was selected for humanization of cH36 HC FR3. The amino acid sequence of a human IgG heavy chain variable domain with a Kabat Database ID No. 000049 was selected for humanization of cH36 HC FR4. The following mutations were made in cH36 HC FR3 to match the amino acid sequence of a human IgG heavy chain variable domain with a Kabat Database ID No. 037010: S76 → T, T77 → S, F80 → Y, H82 → E, N84 → S, T87 → R, D89 → E, S91 → T. One mutations L113 → V was made cH36 HC FR2 to match the amino acid sequence of a human IgG heavy chain variable domain with a Kabat Database ID No. 000049 (see Table 2B for sequence information).

**Table 1.** Comparison of cH36 and Human Light Chain (LC) FR Sequences

**Table 1A**

| Names | LC-FR1 (23 aa) | LC-FR2 (15 aa) |
|---|---|---|
| cH36-LC Human-LC | | |

**Table 1B**

| Names | LC-FR3 (32 aa) | LC-FR4 (10 aa) |
|---|---|---|
| 107 cH36-LC Human-LC | | |

**Table 2.** Comparison of cH36 and Human Heavy Chain (HC) FR Sequences

**Table 2A**

| Names | HC-FR1 (30 aa) | HC-FR2 (14 aa) |
|---|---|---|
| 49 cH36-HC Human-HC | | |

**Table 2B**

| Names | HC-FR3 (32 aa) | HC-FR4 (11 aa) |
|---|---|---|
| cH36-HC Human-HC | | |

Once the decisions on the desired human framework regions were made, the following three techniques were used to achieve the desired amino acid substitutions in both the light and heavy chains: (1) Regular PCR was used for cloning, to introduce cloning or diagnostic restriction endonuclease sites, and to change amino acid residues located at the ends of the variable domains. (2) PCR-based mutagenesis was used to change multiple amino acid residues at a time, especially when these residues were in the middle of the variable domains. (3) Site-directed mutagenesis was used to introduce one or two amino acid substitutions at a time. Site-directed mutagenesis was done following the protocol described in Stratagene's "QuickChange Site-Directed Mutagenesis Kit" (Catalog #200518).

After each step, the partially humanized clones were sequenced and some of these variable domains were later cloned into expression vectors. The plasmid tKMC180 was used to express LC mutants, and the pJRS 355 or pLAM 356 vector was used to express HC mutants as IgG1 or IgG4, respectively. Some of these clones were then combined and expressed transiently in COS cells to determine the expression levels by ELISA.

The final fully humanized forms of the anti-TF heavy and light variable domains were cloned into what is sometimes referred to herein as a "mega vector" and transfected into CHO and NSO cells for IgG expression. Stable cell lines were then used to produce amounts of humanized anti-TF sufficient for analysis. The resulting humanized versions are 100% human in origin (when the CDR sequences are not considered). The humanized IgG4 kappa version is designated hFAT (humanized IgG Four Anti-Tissue Factor antibody) and the IgG1 kappa version is designated hOAT (humanized IgG One Anti-Tissue Factor antibody). These fully humanized versions of cH36 are intended for treating chronic indications, such as thrombosis, cancer and inflammatory diseases.

### C. Generation of Humanized Anti-TF Antibody Heavy Chain

1. PCR amplification and cloning into pGem T-easy of anti-TF mAb cH36 heavy chain (HC) variable domain were performed using plasmid pJAIgG4TF.A8 (an expression vector for chimeric H36) as template and primers TFHC1s2 and TFHC1as2. Primer TFHC1s2 introduced a *BsiW*1 site upstream of the initiation codon and also an amino acid change **E1** to Q in framework (FR) 1. Primer TFHC1 as introduced an amino acid change **L113** to V in FR4. This step resulted in the construct **HC01**.
2. PCR-based mutagenesis using the previous construct (HC01) and the following four primers generated construct **HC02**. Upstream PCR used primers TFHC1s2 and TFHC7as. Downstream PCR used primers TFHC7s and TFHC1as2. PCR using upstream and downstream PCR products as templates and with primers TFHC1s2 and TFHC1as2 yielded **HC02**. The use of primers TFHC7s and TFHC7as introduced two amino acid changes in FR2: **H41** to P and **S44** to G.
3. PCR-based mutagenesis using HC02 as template and the following four primers generated construct **HC03**. Upstream PCR used primers TFHC1s2 and TFHC5as2. Downstream PCR used primers TFHC5s and TFHC1as2. PCR using upstream and downstream PCR products as templates and with primers TFHC1s2 and TFHC1as2 yielded **HC03**. The use of primers TFHC5s and TFHC5as2 introduced three amino acid changes in FR3: **T87** to R, **D89** to E, and **S91** to T. A *Bgl* II site was also introduced at position. 87.
4. PCR amplification was performed using primers TFHC2s and TFHC3as and **HC03** in pGem as template. TFHC2s sits upstream of the cloning site in pGem. TFHC3as sits in framework 3 and introduces two amino acid changes in FR3: **H82** to E and **N84** to S. The resulting PCR band was cloned into pGem and then the proper size insert was digested with *BsiW*1 and *Bgl* II. Cloning of this fragment into **HC03** yields **HC04.**
5. PCR-based mutagenesis using **HC04** as template and the following primers resulted in **HC05.** Upstream PCR used primers TFHC1s2 and TFHC6as. Downstream PCR used primers TFHC6s and TFHC1as2. Mutagenic PCR using upstream and downstream PCR products as templates and with primers TFHC1s2 and TFHC1as2 yielded **HC05.** This step introduced the following amino acid changes in FR3: **S76** to T, **T77** to S, and **F80** toY.
6. PCR-based mutagenesis using **HC05** as template and the following four primers generated **HC06.** Upstream PCR used primers TFHC2s and TFHC2as2. Downstream PCR used primers TFHC3s2 and TFHC1as2. Amplification using TFHC2as2 introduced an amino acid change in FR1: **P9** to G. Primer TFHC3s2 changes **Q19** to R and **T24** to A. PCR using upstream and downstream PCR products as template and with primers TFHC1s2 and TFHC1as2 yielded **HC06.**
7. A point mutation from I to M in position 2 of FR1 was spontaneously introduced during construction of **HC06.** PCR amplification using **HC06** as template and TFHC1s3 and TFHC1as2 as primers, corrected this erroneous substitution and also introduced an amino acid. change in FR1: **Q5** to V. The resulting construct was **HC07.**
8. Construct **HC08** was made by PCR-based mutagenesis using **HC07** as template and the following primers. TFHC2s and TFHC2as3 were used for the upstream product. The downstream product was previously amplified using TFHC1s3 and TFHC1as2 (see step 7). The use of primer-TFHC2as3 introduced two amino acid changes in FR1: **L11** to V and **V12** to K. A spontaneous point mutation resulted in a phenylalanine to leucine (F → L) change at position 64 in CDR2. Further screening and sequencing yielded construct **HC08R1,** which has the correct sequence of F at position 64 in CDR2.
9. Two constructs, **HC11** and **HC12,** were generated by site-directed mutagenesis from **HC07.** Two complementary primers TYHC8sP and TFHC8asP were used along with **HC07** as template to produce **HC11** which contains three amino acid changes in FR1: G9 to P, L11 to V, and V 12 to K. Then, **HC11** was methylated and column purified for the next round of site directed mutagenesis. PCR using **HC11** as a template and the complementary primers TFHC9sL and TFHC0asL generated **HC12** which has a mutation from **V11** to L in FR1.
10. Construct **HC09** was derived from **HC12** by performing PCR using **HC12** as a template and the complementary primers TFHC10sK and TFHC10asK. **HC09** contains an amino acid change: **K12** to V in FR1.
11. Construct **HC10** was made from **HC09.** PCR using **HC09** as a template and the complementary primers LV-1 and LV-2 resulted in the generation of **HC10,** which contains a mutation from **L11** to V in FR1.

After each mutation step, the partially humanized or fully humanized clones were sequenced and some of these variable domains were later cloned into expression vectors. pJRS 355 or pLAM 356 vector was used to express HC mutants fused to Fc of human IgG1 or IgG4.

Figures 3A-D summarize steps 1-11 and shows incremental amino acid changes introduced into FR1-4. Except HC08, all other heavy chain mutants and cH36 contain F at position 64 in CDR2. HC08 has a mutation from F to L at position 64. Figures 4B-D show the heavy chain CDR sequences.

### Primers Used for Heavy Chain Humanization

TFHC1s2
   5'TTTCGTACGTCTTGTCCCAGATCCAGCTGCAGCAGTC 3'
TFHC1as2
   5'AGCGAATTCTGAGGAGACTGTGACAGTGGTGCCTTGGCCCCAG 3'
TFHC7s
   5' GTGAGGCAGAGCCCTGGAAAGGGCCTTGAGTGGATTGG 3'
TFHC7as
   5'CCAATCCACTCAAGGCCCTTTCCAGGGCTCTGCCTCAC3'
TFHC5s
   5'GCATCTCAACAGCCTGAGATCTGAAGACACTGCAGTTTATTTCTGTG3'
TFHC5as2
   5' CTGCAGTGTCTTCAGATCTCAGGCTGTTGAGATGCATGAAGGC 3'
TFHC3as
   5' GTCTTCAGATCTCAGGCTGCTGAGCTCCATGAAGGCTGTGGTG 3'
TFHC2s
   5' TACGACTCACTATAGGGCGAATTGG 3'
TFHC6s
   5'CTGTTGACAAGTCTACCAGCACAGCCTACATGGAGCTCAGCAG3'
TFHC6as
   5'CTGCTGAGCTCCATGTAGGCTGTGCTGGTAGACTTGTCAACAG3'
TFHC2as2
   5'GCACTGAAGCCCCAGGCTTCACCAGCTCACCTCCAGACTGCTGCAGC3'
TFHC3s2
TFHC1s3
   5' TCGTACGTCTTGTCCCAGATCCAGCTGGTGCAGTCTGGAGGTGAGC 3'
TFHC2as3
   5' GCACTGAAGCCCCAGGCTTCTTCACCTCACCTCCAGACTGGACC 3'
TFHC9sL
   5' GCAGTCTGGACCTGAGCTGAAGAAGCCTGGGG 3'
TFHC9asL
   5' CCCCAGGCTTCTTCAGCTCAGGTCCAGACTGC 3'
TFHC8sP
   5' GCTGGTGCAGTCTGGACCTGAGGTGAAGAAGCC 3'
TFHC8asP
   5' GGCTTCTTCACCTCAGGTCCAGACTGCACCAGC3'
TFHC10sK
   5' GCAGTCTGGACCTGAGCTGGTGAAGCCTGGGGCTTC 3'
TFHC10asK
   5' GAAGCCCCAGGCTTCACCAGCTCAGGTCCAGACTGC 3'
LV-1
   5' CAGTCTGGACCTGAGGTGGTGAAGCCTGGG 3'
LV-2
   5' CCCAGGCTTCACCACCTCAGGTCCAGACTG 3'

### D. Generation of Humanized Anti-TF Antibody Light Chain

1. PCR amplification was performed using plasmid pJAIgG4TF.A8 (an expression vector for chimeric H36) as template and primers TFLC1s2.1 and TFLC1as2. This step introduced a cloning site, *Age*I, upstream of the coding region. It also introduced the **L106I** mutation in FR4. This step yielded the construct **LC03.**
2. Site-directed mutagenesis was performed using complementary primers TFLC5s and TFLC5as and LC03 as template. This step introduced the mutation **Q37L** in FR2 and added a PstI site for diagnostic purposes. This new construct is named **LC04.**
3. PCR amplification was performed using **LC04** as template and primers TFHC2s and TFLC2as1. This step generated **Fragment A** that will be used in step 6. This step introduced **Q11L** and **L15V** mutations in FR1.
4. PCR amplification was performed using **LC04** as template and primers TFLC1s2.1 and TFLC1asR. This introduced the *Kpn*I site at the end of LC variable domain. Cloning of this PCR fragment into pGEM yields **pGEM04K** that will be used in step 6.
5. PCR amplification was performed using **LC04** as template and primers TFLC2s and TFLC4as. This step generated **Fragment C** that will be used in step 6. Three mutations **E17D, S18R** in FR1 and **A100Q** in FR4 were introduced in this step.
6. PCR-based mutagenesis using **Fragment A** and **Fragment C** as templates and primers TFHC2s and TFLC4as yielded **Fragment D.** Cloning of **Fragment D** into pGEM04K yielded the construct **LC05.**
7. PCR amplification was performed using pGEM04K as template and primers TFLC1s2.1 and TFLC4as. This step generated **Fragment H,** which is then cloned into pGEM04K. This introduced the **A100Q** mutation in FR4 and the construct is named **LC06.**
8. PCR amplification was performed using **LC06** as template and primers TFLC1s2.1 and TFLC3as. This step generated **Fragment I** that will be used in step 10. This introduced the **K70D** and the **K74T** mutations in FR3.
9. PCR amplification was performed using **LC06** as template and primers TFLC3s2 and TFLC4as. This step generated **Fragment F** that will be used in step 10. This introduced the **A80P** mutation in FR3.
10. PCR using **Fragment I** and **Fragment F** as templates and primers TFLC1s2.1 and TFLC4as yielded **Fragment J.** Cloning of **Fragment J** into pGEM yielded the construct **LC07.**
11. Site-directed mutagenesis was conduced using complementary primers TFLC08sds and TFLC08sdsa and **LC07** as template. This step introduced the mutations **V84A** and **N85T** in FR3. This construct is named **LC08.**
12. The *Age*I to *Eco*O109I fragment from **LC05** containing the mutations **Q11L, L15V, E17D, S18R** and **Q37L** is cloned into **LC08.** This yielded the construct **LC09.**
13. Site-directed mutagenesis was conduced using **LC09** as template and the complementary primers LC105 and LC103. This step introduced the **T85N** mutation in FR3 and yielded the construct **LC10.**
14. Site-directed mutagenesis was conducted using **LC10** as template and the complementary primers LC115 and LC113. This step introduced the **D70K** mutation in FR3. This yielded the construct **LC11.**
15. Site-directed mutagenesis was conducted using **LC11** as template and the complementary primers LC125a and LC123a. This step introduced the **K42Q** mutation in FR2. This yielded the construct **LC12.**

After each mutation step, the partially humanized or fully humanized LC clones were sequenced and some of these variable domains were later cloned into expression vector tKMC180.

### Oligonucleotide Primers Used for Light Chain Humanization

TFLC 1 as2:
   5' TTCGAAAAGTGTACTTACGTTTGATCTCCAGCTTGGTCCCAG 3'
TFLC1s2.1:
   5'ACCGGTGATATCCAGATGACCCAGTCTCC3'
TFLC5s:
   5' GGTTAGCATGGTATCTGCAGAAACCAGGG 3'
TFLC5as:
   5' CCCTGGTTTCTGCAGATACCATGCTAACC 3'
TFHC2s:
   5' TACGACTCACTATAGGGCGAATTGG 3'
TFLC2as 1:
   5' CCACAGATGCAGACAGGGAGGCAGGAGACTG 3'
TFLC1asR:
TFLC2s:
   5' CCTGTCTGCATCTGTGGGAGATAGGGTCACCATCACATGC 3'
TFLC4as:
   5' GATCTCCAGCTTGGTACCCTGACCGAACGTGAATGG 3'
TFLC3as:
   5' GTAGGCTGCTGATCGTGAAAGAA.AAGTCTGTGCCAGATCC 3'
TFLC3s2:
   5' CACGATCAGCAGCCTACAGCCTGAAGATTTTGTAAATTATTACTGTC 3'
TFLC08sds:
   5' GCAGCCTACAGCCTGAAGATTTTGCAACTTATTACTGTCAACAAG 3'
TFLC08sdsa:
   5' CTTGTTGACAGTAATAAGTTGCAAAATCTTCAGGCTGTAGGCTGC 3'
LC105:
   5' CAGCAGCCTACAGCCTGAAGATTTTGCAAATTATTACTGTCAAC 3'
LC103:
   5' GTTGACAGTAATAATTTGCAAAATCTTCAGGCTGTAGGCTGCTG 3'
LC115:
   5' CAGTGGATCTGGCACAAAGTTTTCTTTCACGATCAGCAGC 3'
LC113:
   5' GCTGCTGATCGTGAAAGAAAACTTTGTGCCAGATCCACTG 3'
LC125a:
   5' CTGCAGAAACCAGGGCAATCTCCTCAGCTCCTG 3'
LC123a:
   5' CAGGAGCTGAGGAGATTGCCCTGGTTTCTGCAG 3'

Figure 5A shows the sequence of the human kappa light chain constant domain. Figure 5B shows the human IgG1 heavy chain constant domain. Figure 6A shows the hFAT (IgG4) constant domain sequence. Figure 6B provides the human IgG4 heavy chain constant domain. See also the published U.S. patent application number 20030190705 and references cited therein for additional disclosure relating to the foregoing immunoglobulin constant domain sequences.

### EXAMPLE 2 - Expression and Purification of Humanized anti-TF Antibodies

The partially humanized or fully humanized LC and HC clones were cloned into expression vectors. The plasmid tKMC18 was used to express LC mutants fused to human kappa chain, and pJRS 355 or pLAM 356 vector was used to express HC mutants fused to Fc of human IgG1 or IgG4. Some combinations of the HC and LC clones were then co-transfected into COS cells. The transiently expressed IgGs in COS cells were assayed for the whole IgG production and binding to TF by ELISA. For disclosure relating to these particular vectors see the published U.S. patent application number 20030190705 and references cited therein.

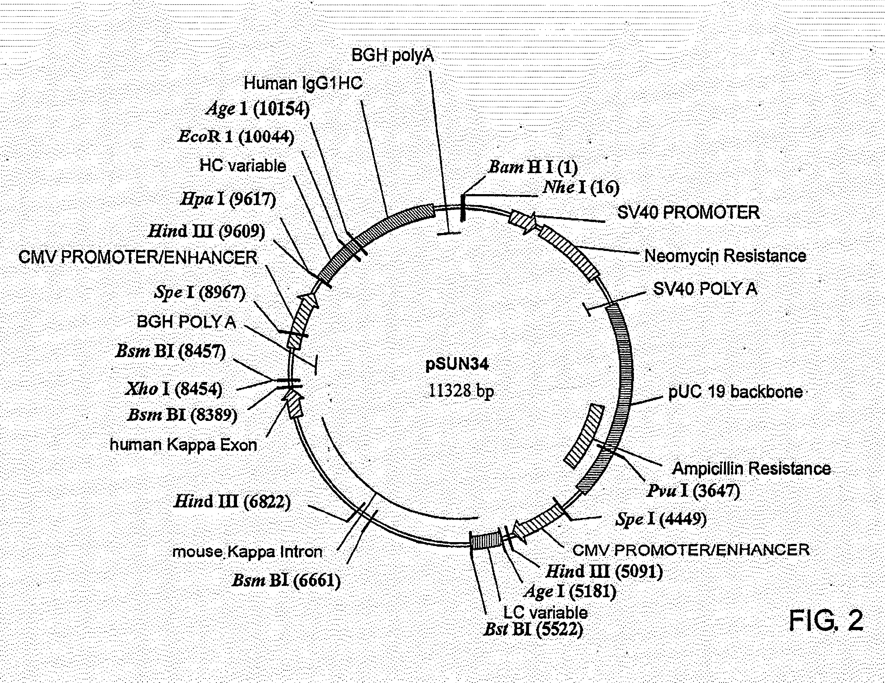

The final fully humanized forms of the anti-TF heavy and light variable domains (combination of HC08 and LC09) were cloned into what is referred to as a Mega expression vector (pSUN34, see Figure 2) and transfected into CHO and NSO cells for IgG expression. Stably transfected cell lines producing the IgG4κ or IgG1κ humanized anti-TF antibody were cloned. The selected stable cell lines were then used to produce amounts of humanized anti-TF sufficient for analysis. The resulting humanized versions are approximately 100% human in origin (when the CDR sequences are not considered). The humanized IgG4 kappa version (produced by pSUN35) is designated hFAT (humanized IgG Four Anti-Tissue Factor antibody) and the IgG1 kappa version (produced by pSUN34) is designated hOAT (humanized IgG One Anti-Tissue Factor antibody). These fully humanized versions of cH36 are intended for treating chronic indications, such as cancer and inflammatory diseases.

One of the NSO cell lines (OAT-NSO-P10A7) that expresses (combination of HC08 and LC09) was thawed and extended in 10 mL of IMDM medium supplemented with 10% FBS in a 15 mL tube and centrifuged. The cell pellet was resuspended in 10 mL of fresh media and passed to a T25 flask and incubated at 37°C in 5% CO₂. In order to prepare a sufficient number of cells to inoculate a hollow fiber bioreactor, the cells were expanded to obtain a total of 6x10⁸ cells. A bioreactor was set up as per manufacturer's instruction manual. The harvested cells were pelleted and resuspended in 60 mL of IMDM containing 35% FBS and injected into the extracapillary space of the bioreactor. Concentrations of glucose and lactate were monitored daily and the harvest material was centrifuged and pooled. The harvested material was tested for anti-TF antibody concentrations by ELISA assay. The pooled sample containing anti-TF antibody (hFAT) were then purified and analyzed as described below.

### A. rProtein A Sepharose Fast Flow Chromatography

Recombinant humanized anti-TF monoclonal antibody consists of two light and two heavy chains. Heavy chain is a fusion of mouse variable domain (unaltered or humanized as described above) and human IgG1 or IgG4 Fc domain, while light chain contains mouse variable domain (unaltered or humanized as described above) and human κ domain. It is well established that human IgG Fc region has high affinity for Protein A or recombinant Protein A (rProtein A).

Harvest pools containing humanized anti-TF antibody (hFAT) were adjusted to pH 8.0 ± 0.1 by adding 0.08 ml of 1 M Tris-HCl, pH 8.0 per ml of sample. Then the sample is filtered through low protein-binding 0.22 micron filters (e.g., Nalgene sterile disposable tissue culture filter units with polyethersulfone membrane from Nalge Nunc International, Cat. No. 167-0020). Following sample application, rProtein A column (from Pharmacia) is washed with 5 bed volumes of 20 mM Tris-HCl, pH 8.0 to remove unbound materials such as media proteins. Since the harvest medium contains high content of bovine serum, a stepwise pH gradient wash was used to remove bovine IgG from the column. The stepwise pH gradient was achieved by increasing the relative percentage of Buffer B (100 mM acetic acid) in Buffer A (100 mM sodium acetate). A typical pH stepwise wash employed 20%, 40%, and 60% Buffer B. Elute the column with 100% Buffer B and collect fractions based on A₂₈₀. The pooled fractions were adjusted to pH 8.5 with addition of 1 M Tris base.

### B. Q Sepharose Fast Flow Chromatography

Anion ion exchange chromatography is very effective in separating proteins according to their charges. The eluted and pH-adjusted sample from rProtein A column was diluted with two volumes of water, and the pH is checked and adjusted to 8.5. The sample was then loaded to a 5 ml (1.6 x 2.5 cm) Q Sepharose Fast Flow equilibrated with 20 mM Tris-HCl, pH 8.5 and the column washed with (1) 5 bed volumes of 20 mM Tris-HCl, pH 8.5; and (2) 4 bed volumes of 20 mM Tris-HCl, pH 8.5 containing 100 mM NaCl. The IgG protein was then eluted with bed volumes of 20 mM Tris-HCl, pH 8.5 containing 500 mM NaCl. The protein peaks were pooled and buffer-exchanged into PBS using ultrafiltration device.

### EXAMPLE 3: Septic Shock Model in Rhesus Monkeys

In this model, septic shock was induced by infusion of live *E. coli*, a gram-negative bacterium (see Taylor et al., J. Clin. Invest. 79:918-825 (1987)) in rhesus monkeys. The shock induced by *E. coli* causes activation both coagulation and inflammation, ultimately leading to death. The ability of an anti-TF antibody of the present disclosure to prolong the survival times of rhesus monkeys treated with live *E. coli* was examined using the rhesus model of septic shock described by Taylor *et al*., *supra.* Rhesus monkeys weighing 3-5 kilograms were fasted overnight before study and immobilized the morning of the experiment with ketamine hydrochloride 14 mg/kg, intramuscularly). Sodium pentobarbital was then administered in the cephalic vein through a percutaneous catheter to maintain a light level of surgical anesthesia (2 mg/kg initially and with additional amounts approximately every 20 to 45 minutes for 6 hours). A femoral vein was exposed aseptically and cannulated in one hind limb for sampling blood and administering gentamicin. Gentamicin was administrated by means of 30-minute intravenous infusions. An infusion of 9 mg/kg was administrated at the end of *E. coli* infusion (t = 2 hours). An infusion of 4.5 mg/kg was administrated 6 hours after *E. coli* infusion. Additional gentamicin (4.5 mg/kg, i.m.) was administrated once daily after day 1 for 3 more days. Each monkey was placed on its side in contact with controlled-temperature heating pads and rectal temperature was monitored. Animals were intubated orally and allowed to breathe spontaneously.

The *E. coli* strain 086:K61H (ATCC 33985) was freshly prepared in less than 12 hours prior to injection. Each monkey received a 2-hour intravenous infusion of *E. coli* at a dose of 4 x 10¹⁰ CFU/kg. Control group monkeys received PBS 30 minutes before infusion of *E. coli.* Treatment group monkeys received a bolus (2-3 minutes) dose of anti-tissue factor antibody (cH36, diluted in PBS if necessary) 30 minutes before infusion of *E. coli* (see Timeline for injection schedule). The percutaneous catheter was used to infuse *E. coli,* PBS and anti-TF antibody.

All monkeys were monitored continuously for 8 hours and observed daily for a maximum of 7 days, for the following: survival time: monitored and recorded hourly; temperature was measured and recorded hourly for the first 8 hours and then once a day for up to 7 days.

Blood samples were collected at the following time points: T = -0.5*, 0**, 1, 2, 4, 6, 24 hours as shown in Table 3 for the analysis of hematological references and inflammatory cytokines. (*T = -0.5, right before injection of cH36 or saline control; **T = 0, right before infusion of *E coli* but 30 min after injection of cH36 or saline control).

**Table 3. The schedule for collecting blood samples.**

| ***Time Point*** | ***Hematology*** | ***Plasma for Analysis*** |
|---|---|---|
| Day 1; t = -0.5 hr | X | X |
| (just prior to test article or vehicle infusion) | | |
| Day 1; t = 0 hour | X | X |
| (30 minutes after treatment, just prior to *E. coli* infusion) | | |
| Day 1; 1 hour (following *E. coli* infusion | X | X |
| Day 1; 2 hour (following *E. coli* infusion) | X | X |
| Day 1; 4 hour (following *E. coli* infusion) | X | X |
| Day 1; 6 hour (following *E. coli* infusion) | X | X |
| Day 1; 24 hour (following *E. coli* infusion) | X | X |
| Volume of whole blood / time point | 1.0 mL | 1.8 mL |
| Anticoagulant | EDTA | Sodium Citrate |

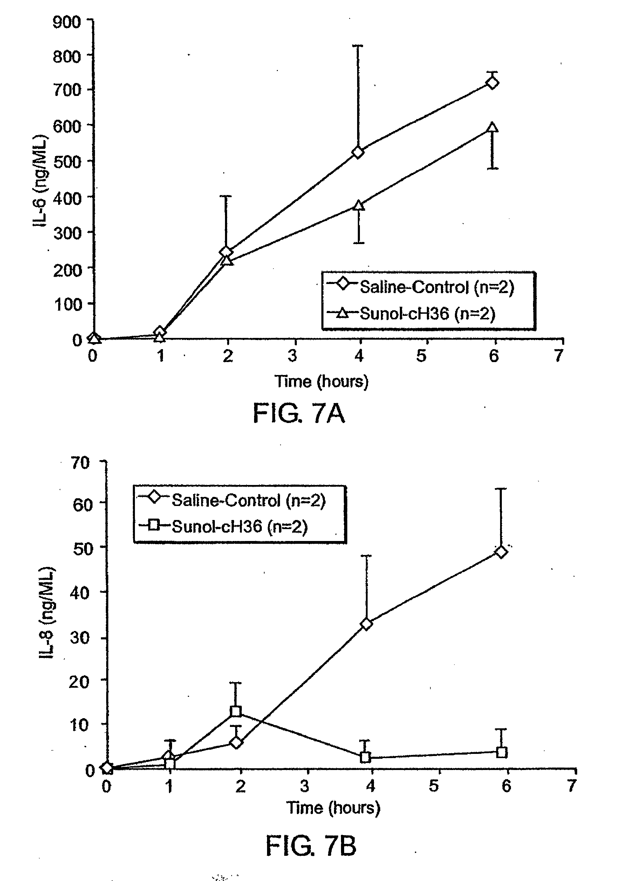

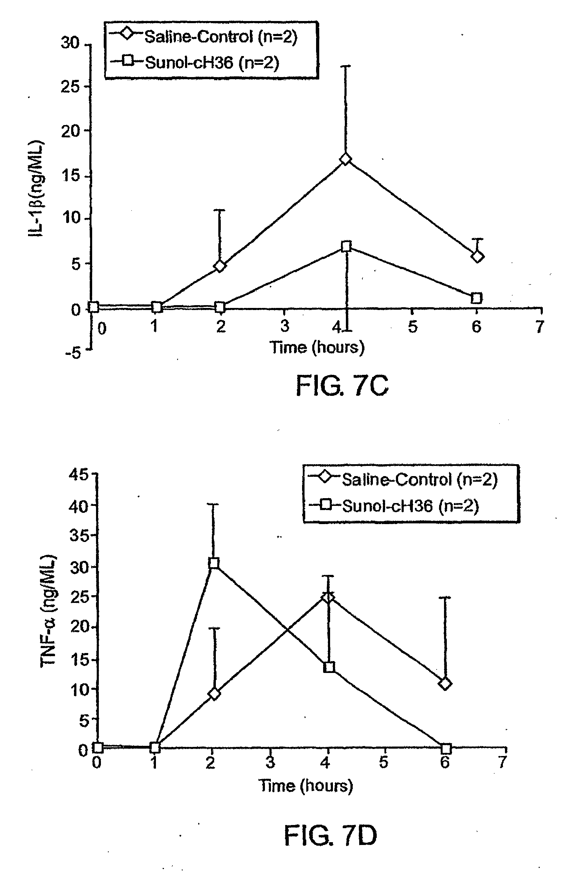

The results of this study are shown in Table 4 and Fig.7A-D. Anti-TF antibody cH36 protected rhesus monkeys very well from E. coli-induced septic shock when administered as a10 mg/kg bolus injected intravenously (Table 4), while attenuating inflammatory cytokines IL-8 and IL-1β, and to a lesser extent IL-6 and TNF-α (Fig. 7A-D).

**Table 4. Protective Effect of cH36 on E coli-induced Septic Shock in Rhesus Monkeys**

| Treatment | Weight (kg) | Sex | Survival Time (hr) | Average Survival Time (hr) |
|---|---|---|---|---|
| | 3.6 | F | 8 | |
| Saline | 4.5 | M | 24 | 16 |
| Sunol-cH36 | 3.1 | F | >168 | |
| (10 mg/kg) | 4.0 | M | 54 | >111 |

### EXAMPLE 4: Acute Lung Injury Model in Baboons

**A.** Acute lung injury is an important cause of morbidity and mortality in sepsis. Patients infected with gram-negative sepsis have a high incidence of acute respiratory distress syndrome and multiple organ failure. It has been shown that blocking tissue factor function with active site-inactivated factor VIIa could limit sepsis-induced acute lung injury and other organ damage in baboons (see Welty-Wolf, K. et al., Am. J. Respir. Crit. Care Med. 164:1988 (2001)). A critical pathophysiological feature of the acute respiratory distress syndrome (ARDS) is local activation of extrinsic coagulation and inhibition of fibrinolysis. As the injury evolves, these perturbations cause deposition of fibrin in the microvascular, interstitial and alveolar spaces of the lung leading to capillary obliteration and hyaline membrane formation. Components of the extrinsic coagulation pathway such as TF, thrombin and fibrin signal alterations in inflammatory cell traffic and increases in vascular permeability. Procoagulants and fibrin also promote other key events in the injury including complement activation, production of proinflammatory cytokines, inhibition of fibrinolysis, and remodeling of the injured lung. It has been established that sepsis-induced TF expression activates the extrinsic coagulation cascade in the lung and leads to a procoagulant environment, which results in fibrin deposition and potentiates inflammation. By blockade of the initiating events of extrinsic coagulation, their effects on proinflammatory events in the lungs and disordered fibrin turnover may be corrected and the evolution of severe structural and functional injury may be averted during experimental ARDS. Recent studies demonstrated that preventing initiation of coagulation at TF-Factor VIIa complex with active site inhibited factor VIIa (FVIIai) or TF pathway inhibitor (TFPI) attenuates fibrin deposition and inflammation in sepsis, thereby limiting acute lung injury (ALI) and other organ damage in baboons.

A model for sepsis-induced ALI model has been established in baboons. See Welty-Wolf, K. et al., Am. J. Respir. Crit. Care Med. 164:1988 (2001). In this model, hyperdynamic cardiovascular and systemic inflammatory responses are preactivated by a priming infusion of killed *E. coli.* After 12 hours, a second dose of live *E. coli* is given to the animals to induce pulmonary and renal failure similar to humans with sepsis and ARDS. Using this model, blockade of TF function by FVIIai and TFPI was shown to attenuate systemic inflammatory responses, decrease fibrin deposition in tissues, and prevented lung and renal injury.

In this model, overnight fasted, adult baboons (*Papio cyanocephalus*) were sedated with intramuscular ketamine (20-25 mg/kg) and intubated. Heavy sedation was maintained with ketamine (3-10 mg/kg/h) and diazepam (0.4-0.8 mg/kg every 2 hours). Animals were mechanically ventilated (21 % O₂) with a volume-cycled ventilator and paralyzed intermittently with pancuronium (4 mg intravenously) before respiratory measurements. An indwelling arterial line and a pulmonary artery catheter were placed via femoral cut down for hemodynamic monitoring. All animals received approximately 10⁹ CFU/kg heat-killed *E. coli* 086:K61H (ATCC 33985) as a 60 min infusion at t = 0 h, 12 h before live *E. coli.* Sepsis was induced at t = 12 h by infusing 10¹⁰ CFU/kg of live *E. coli* in a volume of 50 mL over 60 min. Gentamicin (3 mg/kg i.v.) and Ceftazidime (1 gm i.v.) were administered 60 min after completion of the live *E. coli* infusion. Fluids were given as needed to maintain pulmonary capillary wedge pressure (PCWP) at 8-12 mmHg and to support blood pressure. Dopamine was used for hypotension when mean arterial pressure (MAP) fell below 65 mmHg despite fluids. After 48 h (36 h after the live bacteria infusion) animals were deeply anesthetized and killed by KCl injection. Physiologic parameters including heart rate (HR), temperature, arterial blood pressure, pulmonary artery pressure, ventilator parameters, and fluid intake were recorded every hour. Measurements were obtained every six hours of cardiac output (CO) by thermodilution, central venous pressure (CVP), PCWP, arterial and mixed venous blood gases, oxygen saturation, oxygen content and hemoglobin (Hgb) as reported. Urinary catheter output was measured every six hours and fluid balance calculated as total i.v. fluid intake minus urine output.

Treatment efficacy for each intervention were assessed by comparing the responses of drug-treated animals with vehicle-treated animals using the physiological, histologic, and biochemical endpoints of lung injury listed below:
- Physiological endpoints were evaluated as the alveolar-to-arterial O₂ difference (AaDo₂), pulmonary system compliance (C_{L}), pulmonary artery pressures, pulmonary vascular resistance (PVR) and tissue W/D ratios. Secondary endpoints were fluid volume requirements, serum HCO₃, V_{E} at constant PaCO₂, urine output, creatinine, and systemic DO₂, VO₂ and VCO₂.
- Pathologic endpoints analyzed were gross tissue appearance and qualitative light microscopic analysis, including fibrin deposition, in lung, kidney, adrenal, and other tissues.
- Biochemical endpoints were tissue myeloperoxidase (MPO) levels, total tissue and lavage protein, and lavage LDH. Lung and small bowel edema were measured by wet/dry weights.
Blood samples were drawn at 0, 12, 13, 18, 24, 36, and 48 h. Plasma (from citrated blood) and serum were separated and stored at -80° C. Plasma samples were assayed for interleukin (IL) 6 and IL 8 using ELISA kits (R and D Systems, Inc., Minneapolis, MN).

The experimental protocol is summarized in Table 5.

**Table 5. Experimental Protocol**

| Time (hours) | 0 | 6 | 12 | 14 | 18 | 24 | 30 | 36 | 42 | 48 | | |
|---|---|---|---|---|---|---|---|---|---|---|---|---|
| Heat-killed *E. coli* | X | | | | | | | | | | | |
| Live *E. coli* | | | X | | | | | | | | | |
| Antibiotics | | | | X | | | | | | | | |
| Vehicle or drug | | | | X | X | X | X | X | X | X | | |
| Studies* | X | X | X | X | X | X | X | X | X | X | | |
| Sacrifice | | | | | | | | | | X | | |
| *Studies include serum, plasma, urine and physiology measurements outlined in the detailed methods. | | | | | | | | | | | | |

TF blockade was done using a total antibody dose of 3.5 mg/kg for the cH36 Fab and 5.25 mg/kg for cH36. The intravenous loading dose of test article (1.8 mg/kg for cH36 Fab or 2.7 mg/kg for cH36) was begun 2 hours after infusion of live microorganisms, at the time antibiotics were administered, followed by a constant 34-hour infusion of 50 mcg/kg per hour for cH36 Fab or 75 mcg/kg/hour for cH36.

**Table 6. Experimental Design**

| Group | Treatment | # Animals |
|---|---|---|
| 1 | Sepsis + vehicle | 5 |
| 2 | Sepsis + cH36 Fab (3.5 mg/kg total, 1.8 mg/kg loading, 50 mcg/kg/hr infusion for 34 hr) | 2 |
| 3 | Sepsis + Sunol-cH36 (5.25 mg/kg total, 2.7 mg/kg loading, 75 mcg/kg/hr infusion for 34 hr) | 5 |

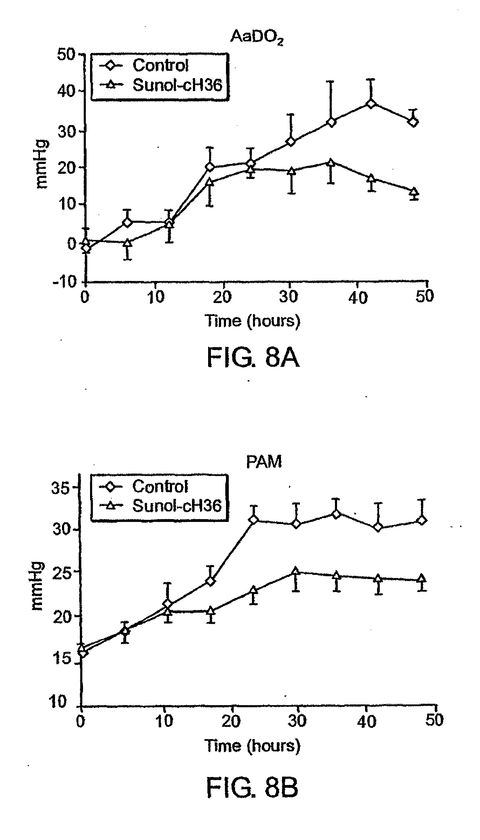

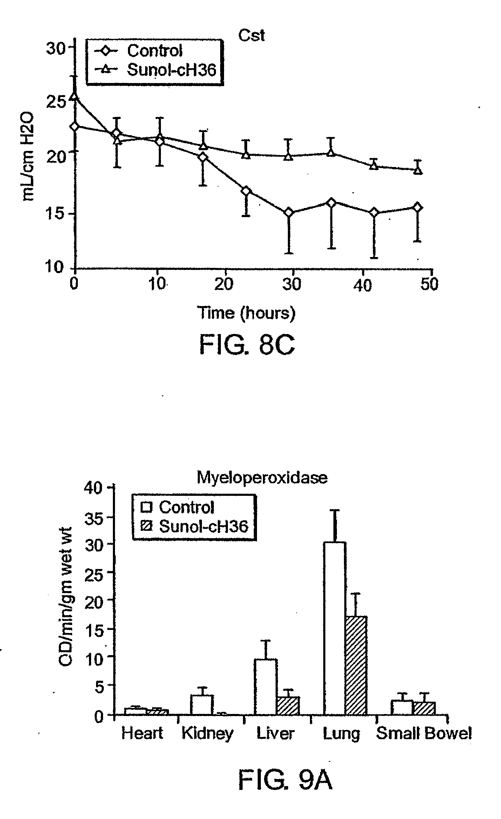

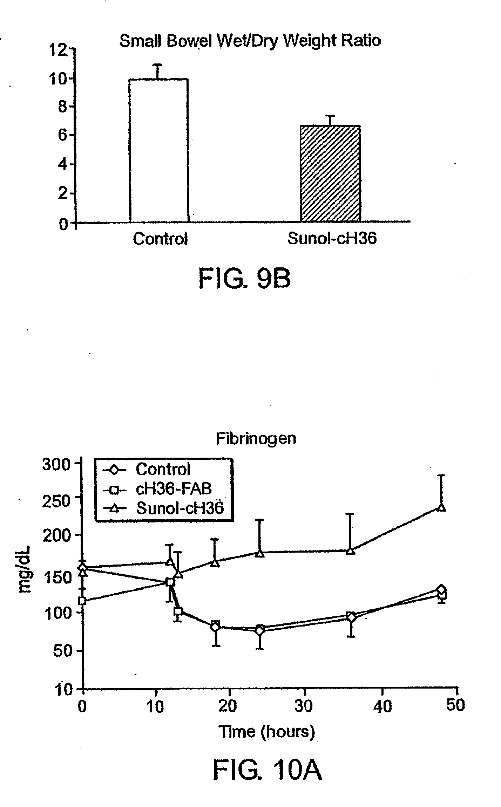

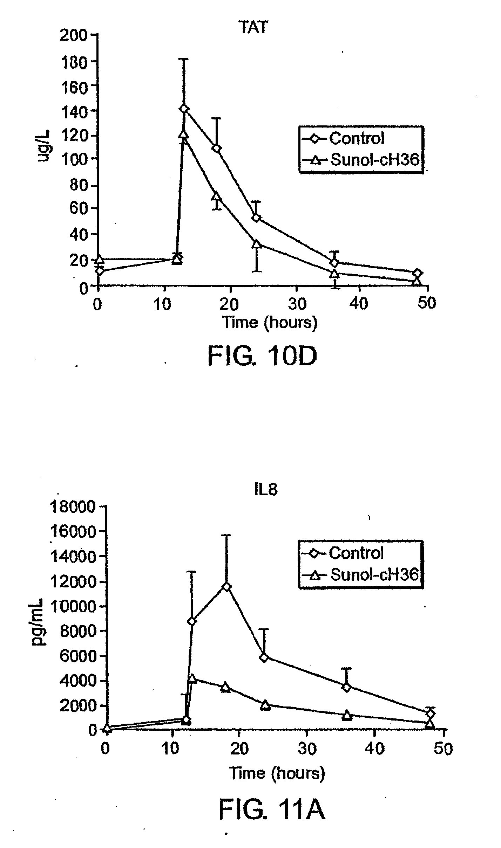

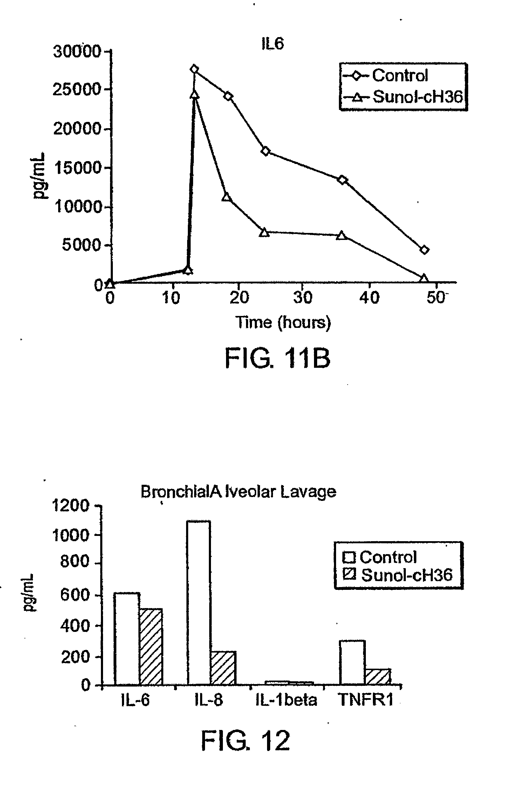

TF blockade with bolus injection of cH36, followed by infusion, attenuated systemic expression of the proinflammatory cytokine IL-8 and IL-6 to a lesser extent (Figs. 11A-B), and provided partial renal and lung protection in baboons challenged with *E. coli*. Interim analysis indicates that cH36 administration attenuates increases in mean pulmonary artery pressure, lung system compliance (Figs. 8A-B) and in the alveolar-arterial oxygen gradient. The kidneys in the animals examined thus far appeared grossly normal at necropsy, the kidney myeloperoxidase levels remained lower (Figure 9A) and urine output was maintained throughout the experiments. The small bowel wet/dry ratio in the treated animals was significantly lower that in the control animals indicative of less edema in the cH36 treated animals (Figure 9B).

These data indicate that coagulation blockade targeting TF with Sunol-cH36 prevents inflammation and the development of organ injury in gram-negative sepsis. These data also show, for the first time, a new strategy for management of ARDS in humans with anticoagulant.

**B.** The following data further show that cH36 and cH36-Fab are useful in preventing lung injury in septic baboons.

Briefly, the objective of this part of the example is to further confirm effects of Sunol-cH36 and cH36-Fab on procoagulant-fibrinolytic balance and inflammation in the lung and relate them to the structural and gas exchange abnormalities in ALI in an experimental sepsis model in baboons. See section A, above.

All baboons (*Papio cyanocephalus*) were mechanically ventilated (21% O₂), anesthetized, and given a dose of heat-killed *E. coli* (1 x 10⁹ CFU/kg) intravenously 12 hours prior to the onset of live *E. coli* sepsis (1-2 x 10¹⁰ CFU/kg). The study design consisted of three groups of baboons as shown in Table 7, below. The baboons in the first group (n=6) were administered vehicle (PBS) and served as controls. The animals in the second group (n=3) were injected with cH36-Fab (3.5 mg/kg total, 1.8 mg/kg bolus loading dose followed by constant infusion of 50 mcg/kg/hr for 34 hours). The third group (n=6) received cH36 (5.25 mg/kg total, 2.7 mg/kg bolus loading dose followed by a constant infusion of 75 mcg/kg/hr for 34 hours). The intravenous loading dose of drug (1.8 mg/kg for cH36-Fab or 2.7 mg/kg for cH36) was started 2 hours after infusion of live microorganisms (at the 14-hour time point) followed by a constant infusion of 50 mcg/kg per hour for cH36-Fab or 75 mcg/kg/hour for cH36 until the end of the experiment at 48 hours. Antibiotics were administered at the 14-hour time point. Treatment efficacy was assessed by comparison of the responses of the treated animals with the controls using physiological, histological and biochemical parameters of lung injury.

**Table 7. Experimental Design**

| **Group** | **Treatment** | **No. of Baboons** |
|---|---|---|
| 1 | **Sepsis + vehicle** | 6 |
| 2 | Sepsis + cH36-Fab (3.5 mg/kg total, 1.8 mg/kg loading, 50 mcg/kg/hr infusion for 34 hr) | 3 |
| 3 | Sepsis + cH36 (5.25 mg/kg total, 2.7 mg/kg loading, 75 mcg/kg/hr infusion for 34 hr) | 6 |

From the foregoing data, it can be concluded that animals treated with cH36 had a less hyperdynamic systemic response to sepsis.

The following Materials and Methods were used as needed to conduct the experiments in this Example. They were also used elsewhere in this disclosure as indicated.

### C. Materials and Methods

Mechanically ventilated (21% O₂), anesthetized baboons (*Papio cyaraocephalus*) were given a dose of heat-killed *E. coli* (1 x 10⁹ CFU/kg) intravenously 12 hours prior to the onset of live *E. coli* sepsis (1-2 x 10¹⁰ CFU/kg). The intravenous loading dose of drug (1.8 mg/kg for cH36-Fab or 2.7 mg/kg for cH36) was started 2 hours after infusion of live microorganisms (14 hours), at the time antibiotics were administered, followed by a constant infusion of 50 mcg/kg per hour for cH36-Fab or 75 mcg/kg/hour for cH36. The total antibody dose was 3.5 mg/kg for the cH36-Fab and 5.25 mg/kg for cH36. Treatment was initiated after the onset of gram-negative sepsis because we have previously shown that TF blockade is effective as a rescue strategy. Because initial studies suggested greater efficacy and no harmful effects of whole antibody compared to Fab, comparisons were made between untreated sepsis controls and septic animals treated with cH36 whole antibody. Experimental groups are as shown in Table 7. Statistical analyses used ANOVA for physiologic data and t test or Mann Whitney U for biochemical and BAL data. Data are expressed as mean ± sem and p values are shown.

Animals were handled in accordance with appropriate guidelines. The animals were divided randomly into treatment and sepsis control groups. After an overnight fast, each animal was sedated with intramuscular ketamine (20-25 mg/kg) and intubated. Heavy sedation was maintained with ketamine (3-10 mg/kg/h) and diazepam (0.4-0.8 mg/kg every 2 hours). Animals were ventilated with a volume-cycled ventilator and paralyzed intermittently with pancuronium (4 mg intravenously) before respiratory measurements. The FiO₂ was 0.21, tidal volume 12 ml/kg, positive end-expiratory pressure 2.5 cm H₂O, and a rate adjusted to maintain an arterial PCO₂ of 40 mmHg. An indwelling arterial line and a pulmonary artery catheter were placed via femoral cut down for hemodynamic monitoring. The animals received gentamicin sulfate (3 mg/kg iv) and ceftazidime (1 gm iv) two hours after the start of live *E. coli* infusion (14 hours). The animals were supported with intravenous volume infusion (Ringer's lactate) at a rate sufficient to maintain the pulmonary capillary wedge pressure (PCWP) at 8 to 12 mmHg. Dopamine was used if needed to maintain mean arterial pressure of 60 mmHg. After 48 h (36 h after the live bacteria infusion) animals were deeply anesthetized and killed by KCl injection. Predefined early termination criteria included refractory hypotension (MAP less than 60 mmHg despite dopamine and adequate PCWP), hypoxemia (need for FIO₂ greater than 40%), or refractory metabolic acidosis (pH < 7.10 with normal PaCO₂). The experimental protocol was the same as that shown in Table 5, above.

Physiological parameters including heart rate (HR), temperature, arterial blood pressure, pulmonary artery pressure, ventilator parameters, and fluid intake were recorded every hour. Measurements were obtained every six hours of cardiac output (CO) by thermodilution, central venous pressure (CVP), PCWP, arterial and mixed venous blood gases, oxygen saturation, oxygen content and hemoglobin (Hgb). Urinary catheter output was measured every six hours and fluid balance calculated as total iv. fluid intake minus urine output.

Blood samples were drawn at 0, 12, 13, 18, 24, 36, and 48 h. Complete blood counts were performed on whole blood (Sysmex-1000 Hemocytometer; Sysmex, Inc., Long Grove, IL). Plasma (from citrated blood) and serum were separated and stored at -80°C. Fibrinogen was measured using an ST4 mechanical coagulation analyzer (Diagnostica Stago, Parsippany, NJ). Prothrombin time (PT) and activated partial thromboplastin time (aPTT) were measured in duplicate, and antithrombin III (ATIII) activity was measured on an MDA coagulation analyzer (Organon Teknika, Durham, NC) with a chromogenic assay and expressed as % of the kit standard. ELISA was used to measure plasma thrombin-antithrombin (TAT) complexes (Dade Behring, Deerfield, IL) in plasma and BAL. cH36 and cH36-Fab levels in blood and BAL were measured by Sunol Molecular (Miramar, FL). Serum samples were assayed for interleukin 1β (IL-1β), IL-6, IL-8, and TNF receptor-1 (TNFR-1) using ELISA kits (R and D Systems, Inc., Minneapolis, MN). Blood creatinine was measured with standard clinical techniques.

Tissues were collected at autopsy as follows: After the experiments the thorax was opened, the left mainstem bronchus ligated, and the left lung removed. BAL was performed on the left upper lobe with 240 mL 0.9% saline. Samples of lung tissue from the left lower lobe were manually inflated and immersed in 4% paraformaldehyde for light microscopy. Four samples were taken at random from the remainder of the left lung for wet/dry weight determination taking care to avoid large vascular and bronchial structures. Additional samples from lung, kidney, liver, small bowel, heart, and adrenal were flash frozen in liquid nitrogen and stored at -80°C. The entire right lung was inflation-fixed for 15 min at 30 cm fixative pressure with 2% glutaraldehyde in 0.85 M Na cacodylate buffer (pH 7.4). Additional tissue from kidney, liver, small bowel, heart, and adrenal was fixed by immersion in 4% paraformaldehye. Four samples of small bowel were selected randomly for wet/dry weight determination.

Myeloperoxidase (MPO) activity and protein concentration of lung homogenates and protein and lactate dehydrogenase (LDH) concentrations of BAL fluid were measured as described (*Am JResp Crit Care Med* 1998;157:938). MPO activity was expressed as a change in absorbance/min/g wet weight tissue. LDH values were expressed in units of activity per liter (U/L).

An important objective of the following Examples was to determine the effects of cH36 and cH36-Fab on procoagulant-fibrinolytic balance and inflammation in the lung. It was also an objective to correlate these parameters to the structural and gas exchange abnormalities in ALI in an experimental sepsis model in baboons.

### D. Results: Treatment with cH36 prevents fibrinogen depletion in baboons with E. coli sepsis.

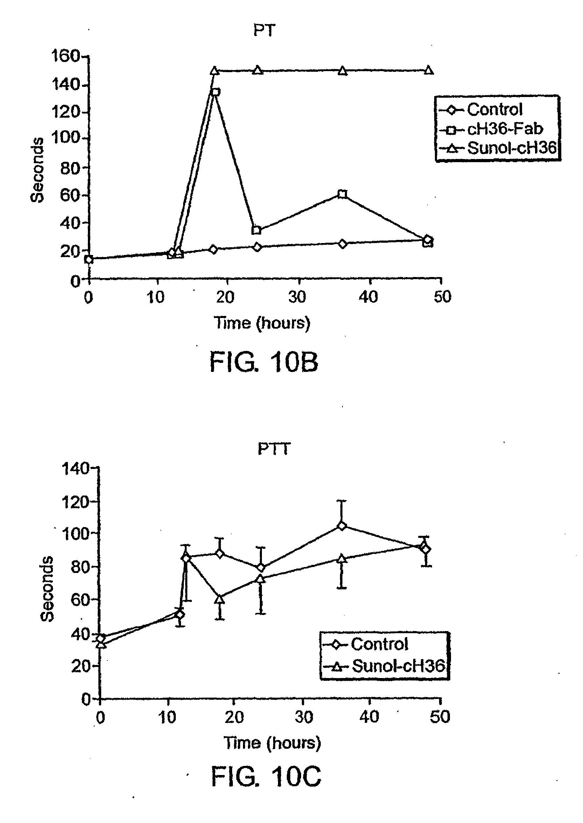

Briefly, cH36 treatment attenuated fibrinogen depletion and TAT complex formation, consistent with inhibition of TF-dependent activation of coagulation. In sepsis controls, fibrinogen decreased to approximately 50% of initial values, but in animals treated with cH36 mean fibrinogen levels did not drop below baseline values (Figure 10A, p<0.01 versus sepsis controls). PT increased in sepsis controls during sepsis due to a progressive coagulopathy, and also increased in treated animals due to pharmacologic effect of the drug infusion. PT and fibrinogen values in the three cH36-Fab treated animals are also shown for comparison. The decreased values in the cH36-Fab compared to whole antibody treated animals suggest that the Fab fragment has a lower affinity for TF (Figure 10A,B). PTT increased in both groups and was not significantly different. TAT complexes increased after infusion of live bacteria in both groups, peaking at 14 h and then decreasing. Peak TAT value was lower and levels decreased more rapidly in animals treated with cH36 (Figure 10D, p<0.01). The difference in TAT complex formation was not due to differences in ATIII levels, as ATIII decreased similarly in the two groups, to 35-40%. of initial values by the end of the experiment.

### E. Results: Treatment with Sunol-cH36 attenuates sepsis-induced acute lung injury

cH36 decreased ALI in baboons with established *E. coli* sepsis, attenuating sepsis-induced abnormalities in gas exchange, pulmonary hypertension, and loss of pulmomary system compliance. These physiologic data are shown in Figures 8A-C. Alveolar arterial oxygen gradient (AaDO₂, mmHg) increased in both groups after infusion of killed bacteria and progressively worsened in the sepsis control group after the onset of live bacterial sepsis at t =12 h. Compared to septic control animals, treatment with cH36 prevented progressive deterioration in gas exchange during sepsis (p = 0.001, Figure 8A). One animal in the sepsis control group required supplemental oxygen after the onset of live bacterial sepsis for progressive hypoxemia. One animal in the cH36 treated group also required oxygen, but only from 18-22 hours, during which oxygenation gradually improved and supplemental O₂ was weaned back to room air. At the end of the experiment the AaDO₂ in that animal had recovered to initial values measured prior to infusion of heat-killed or live bacteria. Sepsis-induced increase in mean pulmonary artery pressure (PAM, mmHg) was attenuated by cH36 (p < 0.0001 vs. untreated sepsis controls, Fig 8B) but there was no difference in pulmonary vascular resistance, suggesting that this was due to effect on cardiac output in the treated animals. cH36 also prevented the decrease in pulmonary system compliance (Cst in mL/cm H₂O) seen in sepsis control animals (p < 0.01, Fig 8C). The PaCO₂ was controlled within the normal range, between 35-45 mmHg in both groups, but was slightly higher in sepsis controls (p=0.03) despite 20% higher minute ventilation (V_{E}, l/min, p=0.015), suggesting cH36 attenuated sepsis-induced increases in dead space (Table 7).

At post-mortem, the lungs from sepsis control animals were dense and hemorrhagic. The gross appearance of the lungs from animals treated with cH36 was improved and in some animals appeared the same as lungs from normal uninjured baboons. Lung wet/dry weights were not significantly different in the two groups, 6.32 ± 0.66 in septic controls compared to 5.57 ± 0.34 in cH36 treated animals (p = NS, normal reference range is 4.6-5.0). BAL protein and LDH were also not significantly different between the two groups. BAL protein was 1.0 ± 0.3 in septic controls compared to 1.0± 0.4 in cH36 treated animals, and LDH was 23.9 ± 10.6 in septic controls compared to 10.6 ± 3 in cH36 treated animals. Neutrophil accumulation, as measured by myeloperoxidase (MPO) activity (OD/min/gm wet wt), was decreased over 40% in cH36 treated animals (p=0.07).

Lung histology showed protection in septic animals treated with cH36. The lungs of sepsis control animals had thickened alveolar septae, patchy alveolar edema and hemorrhage, and intra-alveolar inflammatory cell infiltration with macrophages and PMNs. Lungs of treated animals had improved alveolar septal architecture, decreased alveolar PMN infiltration, less alveolar edema, and no alveolar hemorrhage.

### EXAMPLE 5: Treatment with cH36 improves renal function and reduces organ injury in sepsis

Materials and methods used to perform the present Example have been desribed previously. See eg., Example 4.

Treatment with cH36 improved renal function in sepsis. Urine output was significantly higher after infusion of live *E. coli* in animals treated with cH36 compared to untreated controls (p < 0.001). This was not due to differences in resuscitation because fluid balance and systemic hemodynamics were similar in the two groups. Blood pH and serum [HCO3⁻] were lower in untreated animals (both p < 0.0001), and values were consistent with mixed metabolic and respiratory acidosis in sepsis controls. Serum creatinine was not different in the two groups at the end of the experiments, and there was variability in the treated group due to one animal that was not protected.

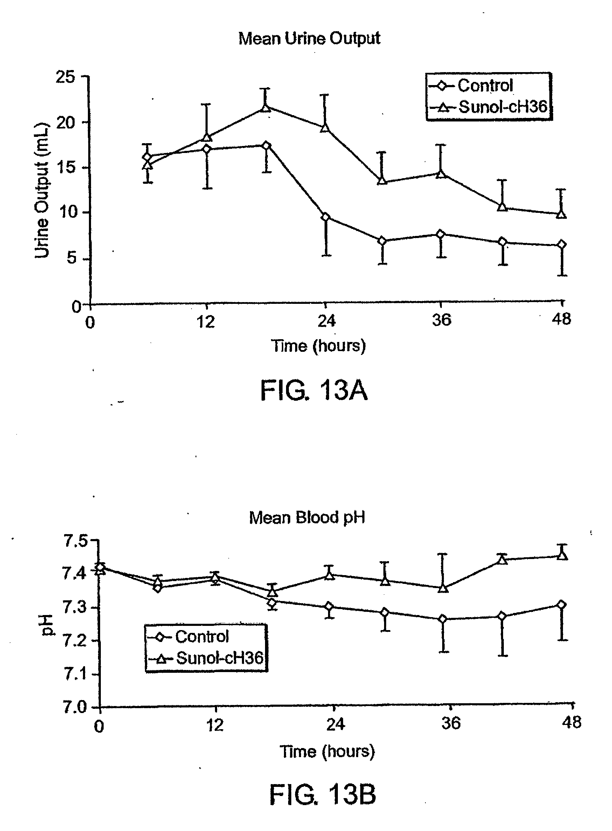

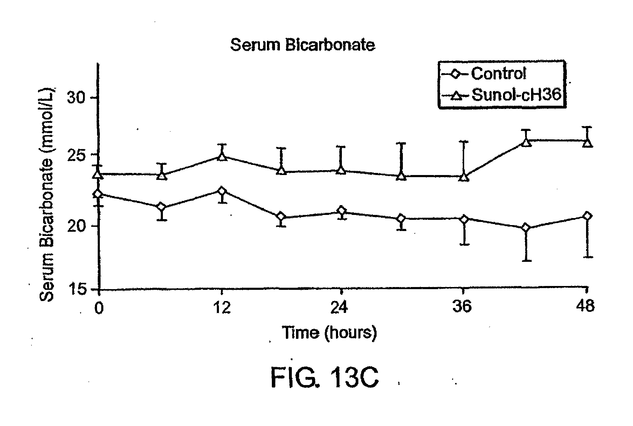

See Figures 13A-C (showing mean urine output (13A), mean blood pH (13B), and serum bicarbonate levels (13C) with control and cH36 antibodies.

Kidneys from untreated animals were swollen and hemorrhagic at post mortem but appeared more normal in cH36 treated animals. H&E stained sections of the kidneys of untreated animals had patchy to extensive areas of acute tubular necrosis (ATN) and glomerular damage. The kidneys of treated animals, except for a few small foci of ATN, showed normal renal architecture. MPO values were significantly decreased in kidneys from treated animals (p=0.01).

Other organ injury was also improved in the cH36 treated animals. Adrenals from untreated animals were swollen and hemorrhagic and small bowel was grossly edematous. In contrast, adrenals and small bowel appeared almost normal in animals treated with cH36. Small bowel wet/dry weights were decreased in septic animals treated with cH36 (6.46 ± 0.62 in Sunol-cH36 treated vs. 9.70 ± 1.05 in untreated sepsis control animals, p = 0.01). Histology confirmed improvement in small bowel edema. Adrenals were also protected from sepsis, with decrease in congestion and hemorrhage, and areas of cellular damage were markedly diminished. In addition to the decreased neutrophil content in the lung and kidney, cH36 treatment significantly decreased PMN content in the liver (pH.05,), and histology showed decreased injury to hepatocytes.

### EXAMPLE 6: cH36 treatment attenuated sepsis-induced anemia

Materials and methods used to perform the present Example have been explained above. See eg., Example 4.

Both groups of animals developed neutropenia, thrombocytopenia and anemia after infusion of live *E. coli* (see Table 8, below). Hemoglobin (Hgb) decreased in both groups but was significantly higher in septic animals treated with cH36 (8.1 ± 0.7 versus 10.8 ± 0.8 g/dL at 48 hours, p < 0.0001). Although platelets declined more rapidly in untreated animals (p<0.0001), the clinical significance of this difference is not clear. All animals developed progressive thrombocytopenia after the infusion of live *E. coli* and mean platelet counts were approximately 30,000 or less in both groups at the end of the experiment. WBC reached a nadir of approximately 1,000-1,500 (x 10³/µL) in both groups two hours after the infusion (t = 14 h) and progressively increased to baseline levels by the end of the experiment (12,680 ± 2,012 in treated vs. 10,500 ± 1,336 in untreated animals, p = 0.07). Two sepsis control animals developed self-limited hematuria. Most animals in both groups had some blood tinged secretions associated with suctioning at some point in the study, but there was no clinical evidence of significant hemorrhage (*i.e*. hematuria, hemoptysis, or bleeding from intravenous or arterial catheter sites) in the cH36 treated animals and no severe or life-threatening bleeding complications occurred in either group.

**Table 8**

| **Time (h)** | | **0** | **12** | **18** | **24** | **36** | **48** | **p value** |
|---|---|---|---|---|---|---|---|---|
| **Hgb (g/dL)** | | | | | | | | |
| | Sepsis | 11.2 ± 0.3 | 10.6 ± 0.3 | 10.4 ± 0.5 | 10.4 ± 0.8 | 8.7 ± 0.8 | 8.1 ± 0.7 | |
| | cH36 | 12.1±0.5 | 11.8 ± 0.4 | 12.2 ± 0.5 | 12.4 ± 0.6 | 10.9 ± 0.8 | 10.8 ± 0.8 | <0.0001 |
| | cH36 Fab | 11.7±0.3 | 11.5 ± 0.5 | 12.5 ± 0.8 | 11.4 ± 0.3 | 11.3 ± 0.4 | 9.6 ± 0.1 | |
| **Platelets** | | | | | | | | |
| | Sepsis | 194 ± 15 | 126 ± 12 | 59 ± 7 | 33 ± 5 | 24 ± 5 | 26 ± 9 | |
| | -cH36 | 206 ± 15 | 167 ± 16 | 110 ± 13 | 64 ± 12 | 40 ± 4 | 30 ± 4 | <0.0001 |
| | cH36 Fab | 197 ± 8 | 146 ± 4 | 87 ± 10 | 31 ± 5 | 21 ± 4 | 25 | |
| **HR (beats/min)** | | | | | | | | |
| | Sepsis | 95 ± 6 | 116 ± 10 | 125 ± 9 | 129 ± 9 | 125 ± 5 | 127 ± 16 | |
| ' | cH36 | 84 ± 3 | 101 ± 4 | 135 ± 13 | 123 ± 11 | 105 ± 12 | 99 ± 9 | <0.01 |
| | cH36 Fab | 75 ± 7 | 89 ± 9 | 107 ± 14 | 109 ± 9 | 98 ± 15 | 98 ± 9 | |
| **MAP (mmHg)** | | | | | | | | |
| | Sepsis | 111 ± 2 | 104 ± 2 | 99 ± 9 | 102 ± 7 | 79 ± 10 | 78 ±13 | |
| | cH36 | 102 ± 3 | 93 ± 3 | 97 ± 5 | 92 ± 6 | 78 ± 9 | 86 ± 5 | NS |
| | cH36 Fab | 108 ± 4 | 85 ± 4 | 89 ± 9 | 78 ± 8 | 89 ± 4 | 63 ± 4 | |
| **CO/kg** | | | | | | | | |
| | Sepsis | 0.16 ± 0.01 | 0.20 ± 0.02 | 0.23 ± 0.04 | 0.22 ± 0.03 | 0.22 ± 0.04 | 0.23 ± 0.02 | |
| | cH36 | 0.15 ± 0.01 | 0.18 ± 0.01 | 0.16 ± 0.02 | 0.16 ± 0.02 | 0.15 ± 0.02 | 0.18 ± 0.01 | <0.0001 |
| | cH36 Fab | 0.12 ± 0.01 | 0.14 ± 0.01 | 0.12 ± 0.01 | 0.15 ± 0.01 | 0.14 ± 0.01 | 0.14 ± 0.02 | |
| **D_{O2}/kg** | | | | | | | | |
| | Sepsis | 23.2 ± 2.2 | 28.1 ± 3.1 | 28.6 ± 4.1 | 26.2 ± 2.0 | 22.7 ± 24.3 | 20.1 ± 1.2 | |
| | cH36 | 23.8 ± 1.8 | 28.0 ± 1.0 | 23.8 ± 2.4 | 24.9 ± 3.5 | 23.1 ± 2.6 | 24.6 ± 2.1 | NS |
| | cH36 Fab | 18.1 ± 1.6 | 21.8 ± 2.1 | 19.5 ± 1.8 | 20.9 ± 2.7 | 21.1 ± 1.8 | 16.4 ± 1.4 | |
| **V_{O2}/kg** | | | | | | | | |
| | Sepsis | 5.4 ± 0.8 | 6.3 ± 1.4 | 6.7 ± 0.6 | 6.8 ± 1.3 | 5.7 ± 0.6 | 5.6 ± 0.7 | |
| | cH36 | 5.6 ± 0.4 | 6.6 ± 0.2 | 6.2 ± 0.4 | 5.6 ± 0.7 | 6.1 ± 0.6 | 6.2 ± 0.3 | NS |
| | cH36 Fab | 4.0 ± 0.3 | 4.1 ± 0.2 | 3.7 ± 0.4 | 4.6 ± 0.3 | 3.7 ± 0.2 | 4.2 ± 0.2 | |
| **SVR*kg** | | | | | | | | |
| | Sepsis | 56602 ± 5459 | 41539 ± 5049 | 35290 ± 4941 | 37788 ± 5675 | 30713 ± 9152 | 26161 ± 5750 | |
| | cH36 | 50833 ± 3782 | 37936 ± 895 | 49953 ± 8998 | 46811 ± 6290 | 37432 ± 3612 | 36058 ± 4362 | *** |
| | cH36 Fab | 69095 ± 2475 | 43890 ± 5700 | 52763 ± 7023 | 39545 ± 7126 | 46570 ± 5101 | 32243 ± 8576 | |
| **PCWP** | | | | | | | | |
| | Sepsis | 10 ± 1 | 11 ± 1 | 10 ± 1 | 11 ± 1 | 12 ± 1 | 11 ± 1 | |
| | cH36 | 10 ± 0 | 10 ± 0 | 9 ± 1 | 9 ± 1 | 11 ± 2 | 9 ± 1 | NS |
| | cH36 Fab | 12 ± 0 | 14 ± 1 | 10 ± 1 | 10 ± 1 | 11 ± 1 | 12 ± 2 | |
| **V_{E}** | | | | | | | | |
| | Sepsis | 3.5 ± 0.3 | 3.5 ± 0.2 | 3.8 ± 0.2 | 4.2 ± 0.2 | 5.0 ± 0.5 | 5.1 ± 0.4 | |
| | cH36 | 3.7 ± 0.1 | 3.9 ± 0.2 | 4.0 ± 0.2 | 4.3 ± 0.2 | 4.4 ± 0.3 | 4.3 ± 0.2 | =0.015 |
| | cH36 Fab | 4.3 ± 0.2 | 4.4 ± 0.2 | 4.5 ± 0.1 | 5.0 ± 0.4 | 6.4 ± 0.8 | 6.2 ± 0.9 | |
| **cH36 (ng/mL)** | | | 15 hr | | | | | |
| | cH36 | 0 | 49.3 ± 5.5 | 51.8 ± 3.3 | 60.2 ± 6.0 | 53.0 ± 5.6 | 58.0 ± 8.3 | |
| | cH36 Fab | 0 | ± | ± | ± | ± | ± | |

Clinical and histological evaluations are performed to confirm that anti-tissue factor injections inhibited the development of rheumatoid arthritis in these mice.

Referring to the foregoing Examples and discussion, it was found that animals treated with cH36 had a less hyperdynamic systemic response to sepsis. Most systemic hemodynamic measurements, including mean arterial pressure (MAP), PCWP, systemic vascular resistance*kg (SVR*kg), VO₂, and DO₂, were not altered by treatment with cH36 (Table 8). Hypotension responded to IV fluids and dopamine infusion in both groups. Nine of the 12 animals survived until the scheduled termination point of the protocol. Overall, septic animals treated with cH36 were less hyperdynamic, without further increases in cardiac output (CO/kg) after the onset of live bacterial sepsis, and had less tachycardia and higher systemic vascular resistance*kg (SVR*kg) at the end of the experiment (Table 7, for instance). In some instances, treated animals needed dopamine and fluid support as did the untreated controls and MAP and SVR were not detectably different between the two groups. Survival is not intended for use as an endpoint as all animals were sacrificed at the 48 hour time point. Three septic animals were treated with cH36-Fab to assess for differences between whole antibody and Fab fragment. As expected, the whole antibody was often more effective then the Fab fragment.

### EXAMPLE 7: Impact of cH36 on pro-inflammatory cytokine levels

Cytokine levels were measured in serum and BAL fluid along lines already described above (eg., Example 5). In the circulation, cH36 treatment attenuated IL-8 (p<0.01, Fig. 12) but had no detectable effect on IL-1β, or TNFR-1. In BAL fluid, cH36 attenuated elevations in IL-6, IL-8 and TNFR-1. BAL cytokine levels are shown in Table 9 below. We also measured soluble thrombomodulin (sTM) and found no differences in treated vs. untreated animals in serum or BAL. The data show that inhibiting coagulation using Sunol-cH36 to block the FX binding site on TF-FVIIa complex attenuates acute lung injury at least in part through effects on proinflammatory cytokine levels in the alveolar compartment. This could be an effect on local cytokine production and/or leakage of proteins from the circulation across the alveolar epithelium.

**Table 9. BAL cytokine levels**

| | Sepsis control | Simol-cH36 | p value | cH36-Fab |
|---|---|---|---|---|
| IL-6 (pg/mL) | 600 ± 269 | 506 ± 522 | 0.05 | 1159 ± 435 |
| IL-8 (pg/mL) | 1081 ± 448 | 224 ± 176 | 0.037 | 1478 ± 755 |
| IL-1β (pg/mL) | 6.9 ± 1.5 | 7.9 ± 3.1. | NS | 8.5 ± 1.8 |
| TNFR1 (pg/mL) | 294 ± 126 | 107 ± 31 | 0.09 | 167 ± 93 |
| sTM (ng/mL) | 1.67 ± 0.54 | 0.9 ± 0.11 | NS | 2.17 ± 0.72 |

Certain results from Examples 4-7 can be summarized as follows:

Most systemic hemodynamic measurements, including mean arterial pressure (MAP), oxygen consumption (VO₂/kg), and oxygen delivery (DO₂/kg), were not altered by treatment with cH36 (Table 8). Mean pulmonary capillary wedge pressure (PCWP) was slightly higher in sepsis control animals (p<0.01) but both groups were within the set parameters of the study. Systemic vascular resistance*kg (SVR*kg) was slightly higher in treated animals (p< 0.05, Table 3). Hypotension responded to IV fluids and dopamine infusion in both groups. Nine of the 12 animals survived until the scheduled termination point of the protocol. Two sepsis control animals died prior to scheduled termination from ALI, with refractory hypoxemia and respiratory acidosis, one at 30 hours and one at 38 hours. One animal in the H36 group was not protected and died at 36 hours of refractory hypotension and metabolic acidosis. In that animal, lack of protection correlated with lower drug levels. Overall, septic animals treated with cH36 were less hyperdynamic, without further increases in cardiac output (CO/kg) after the onset of live bacterial sepsis, and had less tachycardia and higher systemic vascular resistance per kg (SVR*kg) at the end of the experiment (Table 7).

### EXAMPLE 8: Treatment of Baboons with cH36-Fab (Fab fragment)

Three septic animals were treated with cH36-Fab look for any differences between use of whole antibody and Fab fragment. Treatment protocols generally followed procedures already discussed above. Although the group was too small for detailed statistical analyses, the data suggested that the Fab fragment had effect in attenuating sepsis-induced activation of coagulation, with increased TAT values and depletion of fibrinogen similar to septic controls. However, that effect was less pronounced then results achieved with whole antibody. Correspondingly, there was less consistent improvement in gas exchange (AaDO2), pulmonary hypertension (PAM), and lung compliance (Cst) than in animals treated with whole pH36 antibody. Biochemically, lung MPO values were similar to septic controls. Without wishing to be bound to theory, the difference in effect between cH36 and its Fab fragment may be due to the lower affinity of the Fab fragment for TF in the animal model.

### EXAMPLE 9: Anti-Tissue Factor Inhibition of Collagen-Induced Arthritis in Transgenic Mice Expressing Human Tissue Factor

Collagen-induced arthritis (CIA) is an established experimental model of rheumatoid arthritis which is induced in susceptible strains of mice following immunization with type II collagen. In addition to the immune mediated mechanism in the pathogenesis of rheumatoid arthritis, tissue factor initiated activation of the coagulation cascade has also been implicated in the progression of the disease. Fibrin deposition in the affected joints resulting from the activation of coagulation is believed to contribute to synovial thickening and joint inflammation. To determine whether treatment with anti-tissue factor antibodies will prevent development of rheumatoid arthritis mice will be injected with cH36.

To induce CIA 7-12 week old mice are injected intradermally at the base of the tail with 100µg of type II collagen emulsified in Complete Freund's Adjuvant and given a boost injection with 100 µg of type II collagen emulsified in Incomplete Freund's Adjuvant at day 21. Immediately prior to the boost injection mice are given 0.3 mg anti-tissue factor antibody by IV injection (the control group are injected with PBS). Anti-tissue factor antibody injections (0.3 mg) are subsequently given weekly for the duration of the study (the control group are injected with PBS).

Animals are assessed for redness and swelling of the limbs and a clinical score is allocated three times per week. The clinical severity is scored as follows: 1 point for each swollen digit except the thumb (maximum 4), 1 Point for the tarsal or carpal joint, and one point for the metatarsal or metacarpal joint with a maximum score of 6 for a hindpaw and 5 for a forepaw. Each paw is graded individually, the cumulative clinical arthritic score per mouse can reach a maximum of 22 points.

## Claims

1. Use of at least one antibody or fragment thereof, that binds specifically to tissue factor (TF), for the manufacture of a medicament for the prevention or treatment of acute lung injury (ALI) or acute respiratory distress syndrome (ARDS) in a mammal, wherein the antibody has the light chain CDRs 1 to 3 and heavy chain CDRs 1 to 3 as shown by
LASQTIDTWLA;
AATNLAD;
QQVYSS PFT;
DYNVY;
YIDPYNGITIYDQNFKG; and
DVTTALDF, respectively.

2. An antibody or fragment thereof, that binds specifically to tissue factor (TF), for use in the prevention or treatment of acute lung injury (ALI) or acute respiratory distress syndrome (ARDS) in a mammal, wherein the antibody has the light chain CDRs 1 to 3 and heavy chain CDRs 1 to 3 as shown by
LASQTIDTWLA;
AATNLAD;
QQVYSSPFT;
DYNVY;
YIDPYNGITIYDQNFKG; and
DVTTALDF, respectively.

3. An antibody or fragment thereof according to Claim 2 for use according to Claim 2 or use according to Claim 1, wherein the antibody is a chimeric antibody.

4. An antibody or fragment thereof according to Claim 2 for use according to Claim 2 or use according to Claim 1, wherein the antibody has the light chain and heavy chain variable domain amino acid sequences set forth in SEQ ID NOs: 2 and 4, respectively.

5. An antibody or fragment thereof according to Claim 2 for use according to Claim 2 or use according to Claim 1, wherein the antibody comprises the light chain and heavy chain variable domain sequences of an antibody obtained from the cell line H36.D2.B7 that is deposited with the ATCC under accession number HB-12255.

6. An antibody or fragment thereof according to any one of Claims 2 to 5 for use according to any one of Claims 2 to 5 or use according to any one of Claims 1 or 3 to 5, wherein the antibody has an IgG4 isotype human constant domain.

7. An antibody or fragment thereof according to any one of Claims 2 to 6 for use according to any one of Claims 2 to 6 or use according to any one of Claims 1 or 3 to 6, wherein the antibody is a monoclonal antibody.

8. An antibody or fragment thereof according to any one of Claims 2 to 6 for use according to any one of Claims 2 to 6 or use according to any one of Claims 1 or 3 to 6, wherein the antibody or fragment is an Fab, Fab', or F(ab')₂ fragment.

9. An antibody or fragment thereof according to any one of Claims 2 to 6 for use according to any one of Claims 2 to 6 or use according to any one of Claims 1 or 3 to 6, wherein the antibody or fragment is a single-chain immunoglobulin.

10. An antibody or fragment thereof according to any one of Claims 2 to 9 for use according to any one of Claims 2 to 9 or use according to any one of Claims 1 or 3 to 9, wherein the mammal to be treated is a primate.

11. An antibody or fragment thereof according to Claim 10 for use according to Claim 10 or use according to Claim 10, wherein the primate to be treated is a human.

## Patentansprüche

1. Verwendung von mindestens einem Antikörper oder Fragment davon, der bzw. das sich spezifisch an einen Gewebefaktor (TF - Tissue Factor) bindet, zur Herstellung eines Medikaments zur Prävention oder Behandlung von akuter Lungenschädigung (ALI - Acute Lung Injury) oder akutem Atemnotsyndrom (ARDS - Acute Respiratory Distress Syndrome) bei einem Säugetier, wobei der Antikörper die Leichtketten-CDRs 1 bis 3 und die Schwerketten-CDRs 1 bis 3 aufweist, wie jeweils durch Folgendes gezeigt ist:
LASQTIDTWLA;
AATNLAD;
QQVYSSPFT;
DYNVY;
YIDPYNGITIYDQNFKG und
DVTTALDF.

2. Antikörper oder Fragment davon, der bzw. das sich spezifisch an einen Gewebefaktor (TF) bindet, zur Verwendung bei der Prävention oder Behandlung von akuter Lungenschädigung (ALI) oder akutem Atemnotsyndrom (ARDS) bei einem Säugetier, wobei der Antikörper die Leichtketten-CDRs 1 bis 3 und die Schwerketten-CDRs 1 bis 3 aufweist, wie jeweils durch Folgendes gezeigt ist:
LASQTIDTWLA;
AATNLAD;
QQVYSSPFT;
DYNVY;
YIDPYNGITIYDQNFKG und
DVTTALDF.

3. Antikörper oder Fragment davon nach Anspruch 2, zur Verwendung nach Anspruch 2 oder Verwendung nach Anspruch 1, wobei der Antikörper ein chimärer Antikörper ist.

4. Antikörper oder Fragment davon nach Anspruch 2, zur Verwendung nach Anspruch 2 oder Verwendung nach Anspruch 1, wobei der Antikörper die Aminosäuresequenzen der variablen Leichtketten- und Schwerkettendomänen aufweist, die in SEQ ID-Nr.: 2 bzw. 4 dargelegt sind.

5. Antikörper oder Fragment davon nach Anspruch 2, zur Verwendung nach Anspruch 2 oder Verwendung nach Anspruch 1, wobei der Antikörper die Antikörpersequenzen der variablen Leichtketten- und Schwerkettendomänen umfasst, die aus der Zelllinie H36.D2.B7 erhalten werden, die mit der ATCC unter der Zugangsnummer HB-12255 abgelegt wird.

6. Antikörper oder Fragment davon nach einem der Ansprüche 2 bis 5, zur Verwendung nach einem der Ansprüche 2 bis 5, oder Verwendung nach einem der Ansprüche 1 oder 3 bis 5, wobei der Antikörper eine humane Konstantdomäne des IgG4-Isotyps aufweist.

7. Antikörper oder Fragment davon nach einem der Ansprüche 2 bis 6, zur Verwendung nach einem der Ansprüche 2 bis 6, oder Verwendung nach einem der Ansprüche 1 oder 3 bis 6, wobei der Antikörper ein monoklonaler Antikörper ist.

8. Antikörper oder Fragment davon nach einem der Ansprüche 2 bis 6, zur Verwendung nach einem der Ansprüche 2 bis 6, oder Verwendung nach einem der Ansprüche 1 oder 3 bis 6, wobei der Antikörper oder das Fragment ein Fab, Fab', oder F(ab')₂-Fragment ist.

9. Antikörper oder Fragment davon nach einem der Ansprüche 2 bis 6, zur Verwendung nach einem der Ansprüche 2 bis 6, oder Verwendung nach einem der Ansprüche 1 oder 3 bis 6, wobei der Antikörper oder das Fragment ein Einzelketten-Immunglobulin ist.

10. Antikörper oder Fragment davon nach einem der Ansprüche 2 bis 9, zur Verwendung nach einem der Ansprüche 2 bis 9, oder Verwendung nach einem der Ansprüche 1 oder 3 bis 9, wobei das zu behandelnde Säuger ein Primat ist.

11. Antikörper oder Fragment davon nach Anspruch 10, zur Verwendung nach Anspruch 10 oder Verwendung nach Anspruch 10, wobei der zu behandelnde Primat ein Mensch ist.

## Revendications

1. Utilisation d'au moins un anticorps ou un fragment de celui-ci, qui se fixe spécifiquement au facteur tissulaire (TF), pour la fabrication d'un médicament destiné à la prévention ou au traitement d'une lésion pulmonaire aiguë (ALI) ou d'un syndrome de détresse respiratoire aiguë (ARDS) chez un mammifère, l'anticorps possédant les régions déterminant la complémentarité (CDR) de chaîne légère 1 à 3 et les CDR de chaîne lourde 1 à 3 comme il est indiqué par
LASQTIDTWLA ;
AATNLAD ;
QQVYSSPFT ;
DYNVY ;
YIDPYNGITIYDQNFKG ; et
DVTTALDF, respectivement.

2. Anticorps ou fragment de celui-ci, qui se fixe spécifiquement au facteur tissulaire (TF), destiné à une utilisation dans la prévention ou le traitement d'une lésion pulmonaire aiguë (ALI) ou d'un syndrome de détresse respiratoire aiguë (ARDS) chez un mammifère, l'anticorps possédant les CDR de chaîne légère 1 à 3 et les CDR de chaîne lourde 1 à 3 comme il est indiqué par
LASQTIDTWLA ;
AATNLAD ;
QQVYSSPFT ;
DYNVY ;
YIDPYNGITIYDQNFKG ; et
DVTTALDF, respectivement.

3. Anticorps ou fragment de celui-ci selon la revendication 2 destiné à une utilisation selon la revendication 2, ou utilisation selon la revendication 1, l'anticorps étant un anticorps chimérique.

4. Anticorps ou fragment de celui-ci selon la revendication 2 destiné à une utilisation selon la revendication 2, ou utilisation selon la revendication 1, l'anticorps possédant les séquences d'acides aminés de domaine variable de chaîne légère et de chaîne lourde énoncées dans les SEQ ID n^{OS} 2 et 4, respectivement.

5. Anticorps ou fragment de celui-ci selon la revendication 2 destiné à une utilisation selon la revendication 2, ou utilisation selon la revendication 1, l'anticorps possédant les séquences de domaine variable de chaîne légère et de chaîne lourde d'un anticorps obtenu à partir de la lignée cellulaire H36.D2.B7 déposée sous le numéro d'accession ATCC HB-12255.

6. Anticorps ou fragment de celui-ci selon l'une quelconque des revendications 2 à 5 destiné à une utilisation selon l'une quelconque des revendications 2 à 5, ou utilisation selon l'une quelconque des revendications 1 ou 3 à 5, l'anticorps possédant un domaine constant humain d'isotype IgG4.

7. Anticorps ou fragment de celui-ci selon l'une quelconque des revendications 2 à 6 destiné à une utilisation selon l'une quelconque des revendications 2 à 6, ou utilisation selon l'une quelconque des revendications 1 ou 3 à 6, l'anticorps étant un anticorps monoclonal.

8. Anticorps ou fragment de celui-ci selon l'une quelconque des revendications 2 à 6 destiné à une utilisation selon l'une quelconque des revendications 2 à 6, ou utilisation selon l'une quelconque des revendications 1 ou 3 à 6, l'anticorps ou le fragment étant un fragment Fab, Fab', ou F(ab')₂.

9. Anticorps ou fragment de celui-ci selon l'une quelconque des revendications 2 à 6 destiné à une utilisation selon l'une quelconque des revendications 2 à 6, ou utilisation selon l'une quelconque des revendications 1 ou 3 à 6, l'anticorps ou le fragment étant une immunoglobuline monocaténaire.

10. Anticorps ou fragment de celui-ci selon l'une quelconque des revendications 2 à 9 destiné à une utilisation selon l'une quelconque des revendications 2 à 9, ou utilisation selon l'une quelconque des revendications 1 ou 3 à 9, le mammifère à traiter étant un primate.

11. Anticorps ou fragment de celui-ci selon la revendication 10 destiné à une utilisation selon la revendication 10, ou utilisation selon la revendication 10, le primate à traiter étant un humain.
